(19) **Europäisches Patentamt / European Patent Office / Office européen des brevets**

(11) **EP 1 590 666 B1**

(12) **EUROPEAN PATENT SPECIFICATION**

(45) Date of publication and mention
of the grant of the patent:
**09.07.2008 Bulletin 2008/28**

(21) Application number: **03815888.7**

(22) Date of filing: **07.02.2003**

(51) Int Cl.:
*G01N 33/53* (2006.01)     *G01N 33/68* (2006.01)
*C07K 14/47* (2006.01)     *C07K 5/083* (2006.01)
*C07K 5/103* (2006.01)     *A61K 38/06* (2006.01)
*A61K 38/07* (2006.01)     *A61K 38/17* (2006.01)

(86) International application number:
**PCT/US2003/003799**

(87) International publication number:
**WO 2004/072641 (26.08.2004 Gazette 2004/35)**

(54) **COMPOSITIONS AND METHODS FOR ENHANCING APOPTOSIS**

ZUSAMMENSETZUNGEN UND VERFAHREN FÜR VERSTÄRKTE APOPTOSE

COMPOSITIONS ET PROCEDES PERMETTANT D'AUGMENTER L'APOPTOSE

(84) Designated Contracting States:
**AT BE BG CH CY CZ DE DK EE ES FI FR GB GR
HU IE IT LI LU MC NL PT SE SI SK TR**

(43) Date of publication of application:
**02.11.2005 Bulletin 2005/44**

(73) Proprietor: **GENENTECH, INC.
South San Francisco, CA 94080-4990 (US)**

(72) Inventors:
• **FAIRBROTHER, Wayne, J.
Burlingame, CA 94010 (US)**
• **DESHAYES, Kurt
San Francisco, CA 94112 (US)**
• **FISCHER, Saloumeh
Castro Valley, CA 94552 (US)**
• **FLYGARE, John, A.
Burlingame, CA 94010 (US)**
• **FRANKLIN, Matthew, C.
San Francisco, CA 94114 (US)**
• **VUCIC, Domagoj
San Francisco, CA 94110 (US)**

(74) Representative: **Walton, Seán Malcolm et al
Mewburn Ellis LLP
York House
23 Kingsway
London WC2B 6HP (GB)**

(56) References cited:
WO-A-02/26775          WO-A-02/096930
WO-A-03/040172          US-B1- 6 472 172

• WU J-W ET AL: "Structural analysis of a functional DIAP1 fragment bound to grim and hid peptides" MOLECULAR CELL, CELL PRESS, CAMBRIDGE, MA, US, vol. 8, no. 1, July 2001 (2001-07), pages 95-104, XP009018631 ISSN: 1097-2765
• CHAI J ET AL.: "Structural Basis of Caspase-7 Inhibition by XIAP" CELL, vol. 104, 9 March 2001 (2001-03-09), pages 769-780, XP002372300 ISSN: 0092-8674
• VUCIC D ET AL.: "SMAC Negatively Regulates the Anti-apoptotic Activity of Melanoma Inhibitor of Apoptosis (ML-IAP)" THE JOURNAL OF BIOLOGICAL CHEMISTRY, vol. 277, no. 14, 5 April 2002 (2002-04-05), pages 12275-12279, XP002372301
• BLAKE J: "Use of cyclopentyl ester protection for aspartic acid to reduce base catalyszed succinimide formation in solid-phase peptide synthesis" INTERNATIONAL JOURNAL OF PEPTIDE & PROTEIN RESEARCH, vol. 13, no. 4, 1979, pages 418-425, XP008065469 ISSN: 0367-8377
• FRANKLIN M C ET AL: "Structure and function analysis of peptide antagonists of melanoma inhibitor of apoptosis (ML-IAP)" BIOCHEMISTRY, AMERICAN CHEMICAL SOCIETY. EASTON, PA, US, vol. 42, no. 27, 15 July 2003 (2003-07-15), pages 8223-8231, XP002333150 ISSN: 0006-2960

**Description**

FIELD OF THE INVENTION

The present invention is directed to compositions of matter useful for enhancing apoptosis in mammals and to methods of using those compositions of matter for the same.

BACKGROUND OF THE INVENTION.

Apoptosis or programmed cell death is a genetically and biochemically regulated mechanism that plays an important role in development and homeostasis in invertebrates as well as vertebrates. Aberrancies in apoptosis that lead to premature cell death have been linked to a variety of developmental disorders. Deficiencies in apoptosis that result in the lack of cell death have been linked to cancer and chronic viral infections (Thompson et al., (1995) Science 267, 1456-1462).

One of the key effector molecules in apoptosis are the caspases (cysteine containing aspartate specific proteases). Caspases are strong proteases, cleaving after aspartic acid residues and once activated, digest vital cell proteins from within the cell. Since caspases are such strong proteases, tight control of this family of proteins is necessary to prevent premature cell death. In general, caspases are synthesized as largely inactive zymogens that require proteolytic processing in order to be active. This proteolytic processing is only one of the ways in which caspases are regulated. The second mechanism is through a family of proteins that bind and inhibit caspases.

A family of molecules that inhibit caspases are the Inhibitors of Apoptosis (IAP) (Deveraux et al., J Clin Immunol (1999), 19:388-398). IAPs were originally discovered in baculovirus by their functional ability to substitute for P35 protein, an anti-apoptotic gene (Crook et al. (1993) J Virology 67, 2168-2174). IAPs have been described in organisms ranging from *Drosophila* to human. Regardless of their origin, structurally, IAPs comprise one to three Baculovirus IAP repeat (BIR) domains, and most of them also possess a carboxyl-terminal RING finger motif. The BIR domain itself is a zinc binding domain of about 70 residues comprising 4 alpha-helices and 3 beta strands, with cysteine and histadine residues that coordinate the zinc ion (Hinds et al., (1999) Nat. Struct. Biol. 6, 648-651). It is the BIR domain that is believed to cause the anti-apoptotic effect by inhibiting the caspases and thus inhibiting apoptosis. As an example, human X-chromosome linked IAP (XIAP) inhibits caspase 3, caspase 7 and the Apaf-1-cytochrome C mediated activation of caspase 9 (Deveraux et al., (1998) EMBO J. 17, 2215-2223). Caspases 3 and 7 are inhibited by the BIR2 domain of XIAP, while the BIR3 domain of XIAP is responsible for the inhibition of caspase 9 activity.

Melanoma IAP (ML-IAP) is an IAP whose expression is strongly upregulated in melanoma (Vucic et al., (2000) Current Bio 10:1359-1366). Determination of protein structure demonstrated significant homology of the ML-IAP BIR and RING finger domains to corresponding domains present in human XIAP, C-IAP1 and C-IAP2. The BIR domain of ML-IAP appears to have the most similarities to the BIR2 and BIR3 of XIAP, C-IAP1 and C-IAP2, and appears to be responsible for the inhibition of apoptosis, as determined by deletional analysis. Furthermore, Vucic et al., demonstrated that ML-IAP could inhibit chemotherapeutic agent induced apoptosis. Agents such as Adriamycin and 4-tertiary butylphenol (4-TBP) were tested in a cell culture system of melanomas overexpressing ML-IAP and the chemotherapeutic agents were significantly less effective in killing the cells when compared to a normal melanocyte control. The mechanism by which ML-IAP produces an anti-apoptotic activity is through inhibition of caspase 3, 7 and 9. ML-IAP did not effectively inhibit caspases 1, 2, 6, or 8.

Since apoptosis is a strictly controlled pathway with multiple interacting factors, the discovery that IAPs themselves are regulated was not unusual. In the fruit fly Drosophila, the Reaper (rpr), Head Involution Defective (hid) and GRIM proteins physically interact with and inhibit the anti-apoptotic activity of the Drosophila family of IAPs. In the mammal, the proteins SMAC/DIABLO act to block the IAPs and allow apoptosis to proceed. It was shown that during normal apoptosis, SMAC is processed into an active form and is released from the mitochondria into the cytoplasm where it physically binds to IAPs and prevents the IAP from binding to a caspase. This inhibition of the IAP allows the caspase to remain active and thus proceed with apoptosis. Interestingly, sequence homology between the IAP inhibitors shows that there is a four amino acid motif in the N-terminus of the processed, active proteins. This tetrapeptide appears to bind into a hydrophobic pocket in the BIR domain and disrupts the BIR domain binding to caspases (Chai et al., (2000) Nature 406:855-862, Liu et al., (2000) Nature 408:1004-1008, Wu et al., (2000) Nature 408 1008-1012). Further IAP binding peptides are disclosed in WO 02/096930.

Despite the above identified advances in apoptosis research, there is a great need for additional diagnostic and therapeutic agents capable of enhancing apoptosis in a mammal with the goal of inhibiting the progression of cancer. For example, XIAP is expressed in most adult tissues (Duckett et al., (1996) EMBO J. 15, 2685-2694), and therefore, antagonists would sensitize healthy cells to apoptosis by agents used to treat cancers. ML-IAP has been found to be specific for melanoma (Vucic et al., (2000) Current Bio 10:1359-1366), therefore there is utility in developing IAP antagonists that are ML-IAP specific. Accordingly, in the present application, phage display of naïve peptide libraries and synthetic peptide libraries are used to define the interaction of specific amino acids in various peptides as they bind to the structure of ML-IAP. Co-crystallization of selected peptides with ML-IAP, together with peptide SAR (structure-activity-relationship) experiments, determine those peptides that provide increased binding affinity and/or selectivity.

SUMMARY OF THE INVENTION

In one embodiment, the invention provides oligopeptides of sequence $AN_2N_3N_4$, wherein;
$N_2$ is Glu or Asp
$N_3$ is Val, Ile, Leu or (2S, 3S)-3-methylpyrrolidine-2-carboxylic acid [(3S)-methyl -proline]
$N_4$ is homophenylalanine, 4-amino-phenylalanine, 4-phenyl-phenylalanine, 2,2-diphenylethylamine, (1S,2S)-(+)-2-amino-1-phenyl-1,3-propandiol, 3-trifluoromethylphenylethylamine, (1R,2R)-(-)-2-amino-1-phenyl-1,3-propandiol, trans-2-phenylcyclopropylamine, (1R,1S)-(+)-norephedrine, β-methylphenylethylamine, (S)-(-)-2-amino-3-phenyl-1-propanol, (R)-(-)-2-amino-1-phenylethanol, 3-ethoxyphenylethylamine, 5-bromo-2-methoxyphenylethylamine, 3-fluorophenylethylamine, (S)-(+)-α-(methoxymethyl)-phenylethylamine, 3-chlorophenylethylamine, or 2-ethoxyphenylethylamine which bind, preferably specifically, to the above or below described BIR domain, (**B**IR **D**omain **B**inding oligopeptide; henceforth BDB oligopeptide). Optionally, the BDB oligopeptides of the present invention may be conjugated to a growth inhibitory agent or cytotoxic agent such as a toxin, including, for example, a maytansinoid or calicheamicin, an antibiotic, a radioactive isotope, a nucleolytic enzyme, or the like. The BDB oligopeptides of the present invention may optionally be produced in CHO cells or bacterial cells and preferably induce death of a cell to which they bind. For diagnostic purposes, the BDB oligopeptides of the present invention may be detectably labeled, attached to a solid support, or the like.

In other embodiments of the present invention, the invention describes vectors comprising DNA encoding any of the herein described binding oligopeptides. Host cell comprising any such vector are also provided. By way of example, the host cells may be CHO cells, *E. coli* cells, or yeast cells. A process for producing any of the herein described binding oligopeptides is further disclosed and comprises culturing host cells under conditions suitable for expression of the desired oligopeptide and recovering the desired oligopeptide from the cell culture.

In a still further embodiment, the invention concerns a composition of matter comprising a chimeric BDB oligopeptide as described herein. Optionally, the chimeric BDB oligopeptide concerns a BDB oligopeptide fused to a peptide or small molecule to facilate the transport of the BDB oligopeptide across the cell membrane. Optionally, the invention concerns a chimeric BDB oligopeptide, a BDB oligopeptide as described herein in combination with a carrier. Optionally, the carrier is a pharmaceutically acceptable carrier.

In yet another embodiment, the invention concerns an article of manufacture comprising a container and a composition of matter contained within the container, wherein the composition of matter may comprise a chimeric BDB polypeptide as described herein or a BDB oligopeptide as described herein. The article may further optionally comprise a label affixed to the container, or a package insert included with the container, that refers to the use of the composition of matter for the therapeutic treatment or diagnostic detection of a tumor.

Another embodiment of the present invention is directed to the use of a chimeric BDB polypeptide as described herein or a BDB oligopeptide as described herein for the preparation of a medicament useful in the treatment of a condition which is responsive to the chimeric BDB polypeptide or BDB oligopeptide.

Another embodiment of the present invention is directed to the use of a chimeric BDB polypeptide as described herein or a BDB oligopeptide as described herein for the preparation of a medicament useful in the treatment of a condition which is responsive to the chimeric BDB polypeptide or BDB oligopeptide, where the BIR domain is specifically that of ML-IAP.

1. Additional Embodiments

Another embodiment of the present invention is directed to a method for inhibiting the growth of a cell that expresses an IAP polypeptide comprising a BIR domain, wherein the method comprises contacting the cell in vitro with a BDB oligopeptide that binds to the IAP polypeptide, and wherein the binding of the BDB oligopeptide to the IAP polypeptide causes inhibition of the growth of the cell expressing the IAP polypeptide. In preferred embodiments, the cell is a cancer cell and binding of the BDB oligopeptide to the IAP polypeptide allows apoptosis of the cell expressing the IAP polypeptide. Optionally, the IAP polypeptide is ML-IAP.

BDB oligopeptides employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme. The BDB oligopeptides employed in the methods of the present invention may optionally be produced in CHO cells, bacterial cells or synthetically synthesized.

Further described is a method of therapeutically treating a mammal having a cancerous tumor comprising cells that express a IAP polypeptide, wherein the method comprises administering to the mammal a therapeutically effective amount of a BDB oligopeptide that binds to the IAP polypeptide, thereby resulting in the effective therapeutic treatment of the tumor. Optionally, the IAP polypeptide is ML-IAP. BDB oligopeptides employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme. The BDB oligopeptides employed in the methods of the present invention may optionally be produced in CHO cells, bacterial cells or synthetically synthesized.

Yet another embodiment discloses a method of determining the presence of a IAP polypeptide in a sample suspected of containing the IAP polypeptide, wherein the method comprises exposing the sample to a BDB oligopeptide that binds to the IAP polypeptide and determining binding of the BDB oligopeptide to the IAP polypeptide in the sample, wherein the presence of such binding is indicative of the presence of the IAP polypeptide in the sample. Optionally, the sample may contain cells (which may be cancer cells) suspected of expressing the IAP polypeptide. The BDB oligopeptide employed in the method may optionally be detectably labeled, attached to a solid support.

A further embodiment describes a method of diagnosing the presence of a tumor in a mammal, wherein the method comprises detecting the level of expression of a gene encoding a IAP polypeptide (a) in a test sample of tissue cells obtained from said mammal, and (b) in a control sample of known normal non-cancerous cells of the same tissue origin or type, wherein a higher level of expression of the IAP polypeptide in the test sample, as compared to the control sample, is indicative of the presence of tumor in the mammal from which the test sample was obtained.

Another embodiment deals with a method of diagnosing the presence of a tumor in a mammal, wherein the method comprises (a) contacting a test sample comprising tissue cells obtained from the mammal with a BDB oligopeptide that binds to a IAP polypeptide and (b) detecting the formation of a complex between the BDB oligopeptide and the IAP polypeptide in the test sample, wherein the formation of a complex is indicative of the presence of a tumor in the mammal. Optionally, the BDB oligopeptide employed is detectably labeled, attached to a solid support, or the like, and/or the test sample of tissue cells is obtained from an individual suspected of having a cancerous tumor.

Yet another embodiment describes a method for treating or preventing a cell proliferative disorder associated with altered, preferably increased, expression or activity of a IAP polypeptide, the method comprising administering to a subject in need of such treatment an effective amount of an antagonist of a IAP polypeptide. Preferably, the cell proliferative disorder is cancer and the antagonist of the IAP polypeptide is a BDB oligopeptide. Effective treatment or prevention of the cell proliferative disorder may be a result of direct killing or growth inhibition of cells that express a IAP polypeptide or by antagonizing the IAP polypeptide and rendering it sensitive to apoptosis inducing agents.

Yet another embodiment of the present invention is directed to a method of binding a BDB oligopeptide to a cell that expresses a IAP polypeptide, wherein the method comprises contacting a cell in vitro that expresses a IAP polypeptide with said BDB oligopeptide under conditions which are suitable for binding of the BDB oligopeptide to said IAP polypeptide and allowing binding there between.

Other embodiments of the present invention are directed to the use of a BDB oligopeptide, in the preparation of a medicament useful for (i) the therapeutic treatment or diagnostic detection of a cancer or tumor, or (ii) the therapeutic treatment or prevention of a cell proliferative disorder.

Another embodiment of the present invention is directed to a method for inhibiting the growth of a cancer cell in vitro wherein the growth of said cancer cell is at least in part dependent upon the growth potentiating effect(s) of an IAP polypeptide, wherein the method comprises contacting the IAP polypeptide with a BDB oligopeptide that binds to the IAP polypeptide, thereby antagonizing the apoptosis inhibiting activity of the IAP polypeptide and, in turn, inhibiting the proliferation of the cancer cell. Preferably the proliferation of the cancer cell is completely inhibited. Even more preferably, binding of the BDB oligopeptide to the IAP polypeptide induces the apoptosis of the cancer cell. BDB oligopeptides employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme. The BDB oligopeptides employed in the methods of the present invention may optionally be produced in CHO cells, bacterial cells or synthetically synthesized.

Yet another embodiment describes a method of therapeutically treating a tumor in a mammal, wherein the growth of said tumor is at least in part dependent upon the growth potentiating effect(s) of a IAP polypeptide, wherein the method comprises administering to the mammal a therapeutically effective amount of a BDB oligopeptide that binds to the BIR domain, thereby antagonizing the growth potentiating activity of said IAP polypeptide and resulting in the effective therapeutic treatment of the tumor. BDB oligopeptides employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme. The BDB oligopeptides employed in the methods of the present invention may optionally be produced in CHO cells, bacterial cells or synthetically synthesized.

Further described is a method of use of the crystal structure of ML-IAP BIR domain, wherein said crystal structure is used to identify contact residues between the BIR domain and a potential inhibitor. Such contact residues and neighboring residues are candidates for substitution according to the techniques elaborated herein. Once such variants are generated, the panel of variants is subjected to screening as described herein and IAP inhibitors with superior properties in one or more relevant assays may be selected for further development.

## BRIEF DESCRIPTION OF THE DRAWINGS

FIGURE 1: Amino acid sequence alignment of the nine N-terminal residues of the small subunit of active human, murine, and *Xenopus* caspase-9, active mammalian SMAC/DIABLO, HtrA2/Omi, and *Drosophila* Reaper, Hid, Grim, and Sickle.

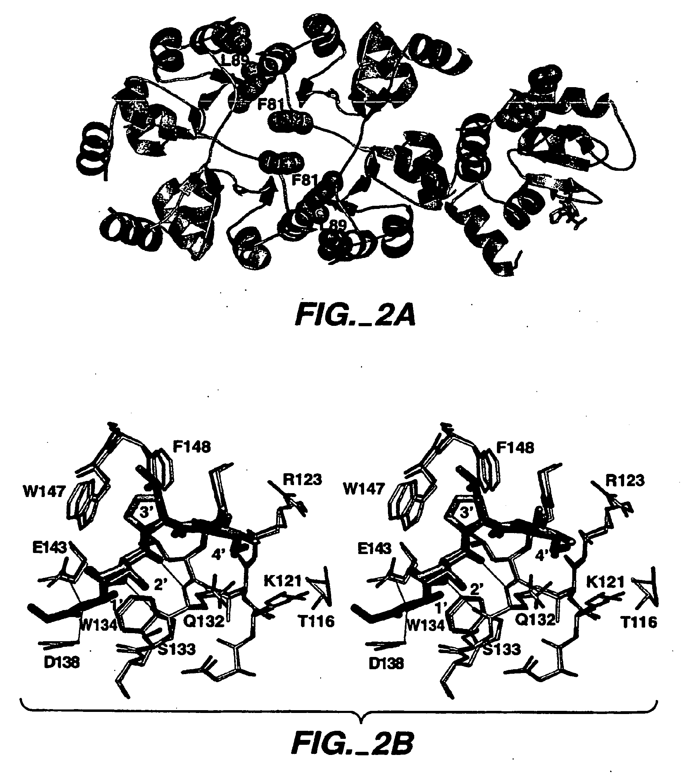

FIGURE 2A-B: (A) Schematic representation of the five copies of ML-IAP-BIR in the asymmetric unit of the crystals of the ML-IAP-BIR/AVPIAQKSE complex showing the interdomain interactions. The hydrophobic interface residues Phe81 and Leu89 are shown in CPK rendering. The SMAC-based peptide bound to protomer E is shown in stick rendering. (B) Stereoview showing a least-squares superposition of the peptide binding site occupied by the peptide AVPIAQKSE (SEQ ID NO: 1) or residues Gly72-Ala73-Thr74-Leu75-Ser76 from a symmetry related protein molecule. Hydrogen-bonds between the peptide and protein are indicated by thin lines.

FIGURE 3: Amino acid preferences (corrected for codon bias) for the binding peptides selected on phage against XIAP-BIR2, XIAP-BIR3 and ML-IAP-BIR.

FIGURE 4: Overall structure of ML-IAP-BIR in complex with the SMAC-based peptide AVPIAQKSE (SEQ ID NO: 1). The secondary structure comprises five $\alpha$-helices (residues 87-92, 105-110, 140-147, 152-158, and 160-168) and a three-stranded $\beta$-sheet (residues 113-115, 122-124, and 130-132). Also shown is a zinc atom chelated by three Cys and one His residues (Cys124, Cys127, Cys151, and His 144).

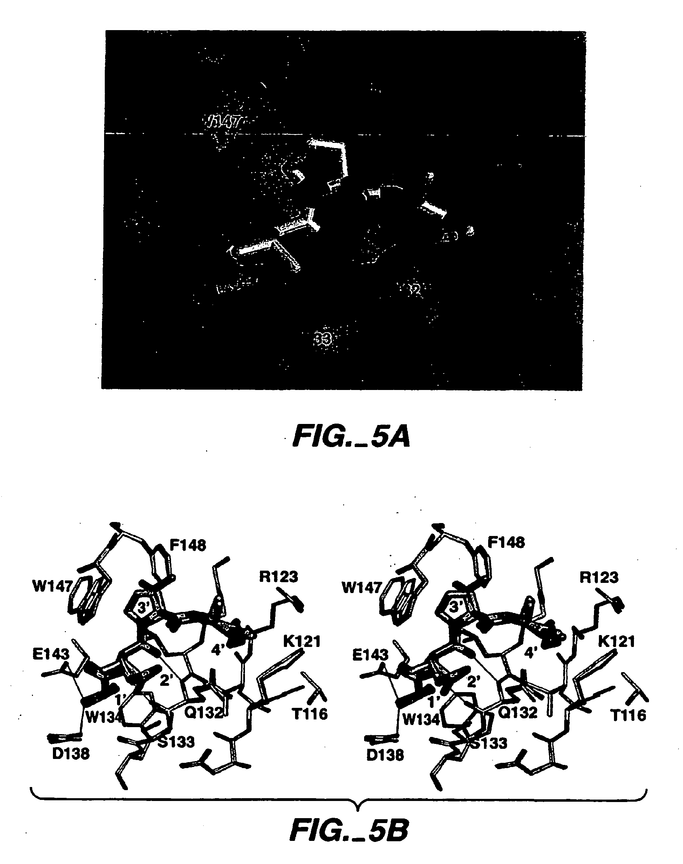

FIGURE 5A-C: (A) Solvent-accessible surface representation of the peptide-binding site of ML-IAP-BIR is shown. The bound SMAC-based peptide is also shown. (B) Stereoview showing least-squares superpositions (using protein residues 78-170 and peptide residues 1'-4') of the peptide binding sites of complexes between ML-IAP-BIR and the peptides AVPIAQKSE (SEQ ID NO:1), AEAVPWKSE (SEQ ID NO:19) and AEVVAVKSE (SEQ ID NO:10). Hydrogen-bonds between the peptide and the protein are indicated by thin lines. (C) Structure-based sequence alignment of ML-IAP-BIR, XIAP-BIR3 and XIAP-BIR2. The secondary structure of ML-IAP-BIR is indicated above the sequence. Residues indicated with an asterisk are within 4 Å of the SMAC-based peptide in the ML-IAP-BIR/AVPIAQKSE complex structure.

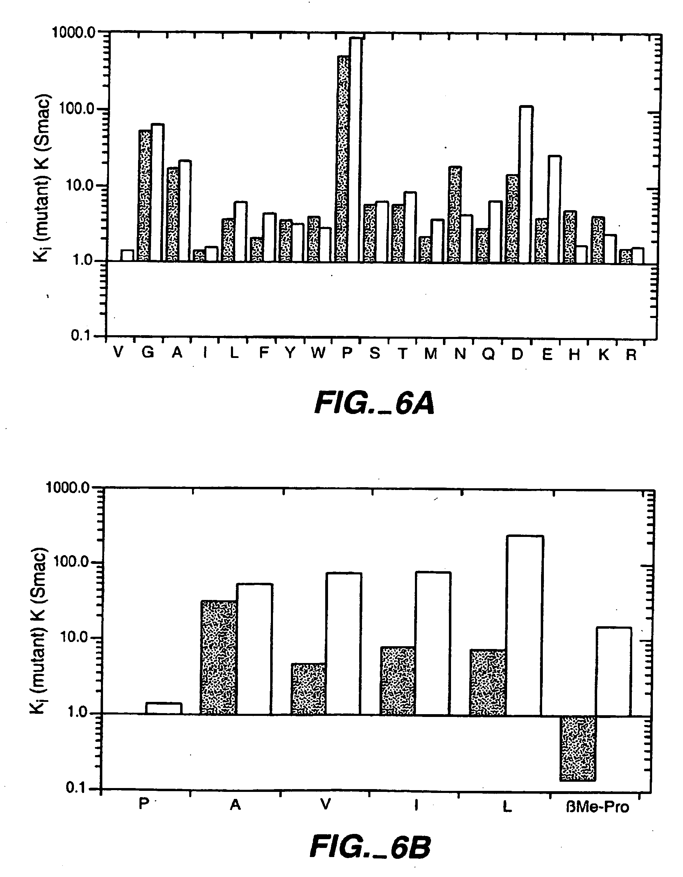

FIGURE 6A-C: Log plots of relative $K_i$ [$K_i$(mutant peptide/protein)/$K_i$(SMAC peptide/ML-IAP-BIR] for SMAC-based peptides substituted at residues 2' (A), 3' (B), or 4"(C) binding to ML-IAP-BIR (black) or XIAP-BIR3 (gray). $K_i$ values were determined from a fluorescence polarization competition assay using either SMAC-FAM or Hid-FAM probes (as described in Materials and Methods). $K_d$ values for these probes are 0.15 and 0.038 $\mu$M, respectively, for binding to ML-IAP-BIR, and 0.26 and 0.11 $\mu$M, respectively, for binding to XIAP-BIR3. Non-natural amino acids are indicated as follows: $\beta$Me-Pro, (3S)-methyl-proline; X1, 2-naphthylalanine; X2, phenylalanine-4-sulfonic acid; X3, 4-nitro-phenylalanine; X4, 4-amino-phenylalanine; X5, 3-methoxy-phenylalanine; X6, cyclohexylalanine; X7, cyclopentylalanine; X9, 3,5-dibromo-tyrosine; X10, 4-iodo-phenylalanine; X12, homophenylalanine; X13, 4-ketophenyl-phenylalanine; X14, 4-phenyl-phenylalanine.

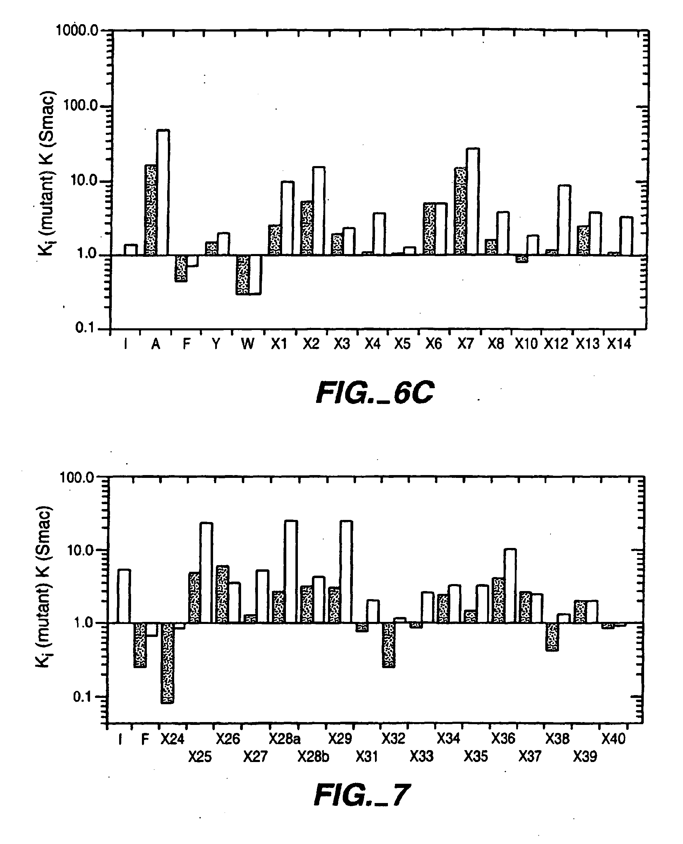

FIGURE 7: Log plot of relative $K_i$ [$K_i$(substituted peptide/protein)/ $K_i$(AVPI/ML-IAP-BIR)] for SMAC-based peptides substituted at residue 4' with selected amino acids or phenylethylamines binding to ML-IAP-BIR (black) or XIAP-BIR3 (gray). $K_i$ values were determined from a fluorescence polarization assay using a Hid-FAM probe (as described ). The phenylethylamine derivatives are indicated as follows: X24, 2,2-diphenylethylamine; X25, (1S, 2S)-(+)-2-amino-1-phenyl-1,3-propandiol; X26, 3-trifluoromethylphenylethylamine; X27, (1R,2R)-(-)-2-amino-1-phenyl-1,3-propandiol; X28a, trans-2-(1R, 2S)-2-phenylcyclopropyl-1-amine; X28b, trans-(1S,2R)-(-)-2-amino-1-phenylcyclopropyl-1-amine; X29, (1R,1S)-(+)-norephedrine; X31, $\beta$-methylphenylethylamine; X32, (S)-(-)-2-amino-3-phenyl-1-propanol; X33, (R)-(-)-2-amino-1-phenylethanol; X34, 3-ethoxyphenylethylamine; X36, 5-bromo-2-methoxyphenylethylamine; X37, 3-fluorophenylethylamine; X38, (S)-(+)-a$\alpha$-(methoxymethyl)-phenylethylamine; X39, 3-chlorophenylethylamine; X40, 2-ethoxyphenylethylamine

FIGURE 8: Solvent-accessible surface representation of the peptide-binding site from the crystal structure of ML-IAP-BIR in complex with AVPX24 (where X24 corresponds to 2,2-diphenylethylamine). The bound peptide-phenylethylamine derivative is shown in stick representation.

FIGURE 9A-B $^{15}$N,$^{1}$H-HSQC spectra of (A) 0.6 mM ML-IAP-BIR and (B) 0.6 mM Phe81Glu/Leu89Asp mutant ML-IAP-BIR, in 50 mM potassium phosphate (pH 7.2), 150 mM sodium chloride solution acquired at 25°C on a Bruker DRX-600 NMR spectrometer.

FIGURE 10A-E: Shows the structure of dipeptide isosteres capable of binding to and antagonizing the IAP proteins. Figure 10A shows the sequence A(Xaa)IAQKSE where Xaa is (3S)-3-amino-1-carboxymethyl-caprolactame, or any of the dipeptide isosteres as shown in Figures 10B-10E.

FIGURE 11: SMAC-like peptides block the anti-apoptotic activity of ML-IAP. Cells transfected with IAPs showed a reduction in apoptosis when treated with adriamycin. Addition of BDB oliopeptides in combination with adriamycin increase the amount of apoptosis.

## DETAILED DESCRIPTION OF THE PREFERRED EMBODIMENTS

Definitions.

"Isolated," when used to describe the various oligopeptides disclosed herein, means oligopeptide that has been identified and separated and/or recovered from a component of its natural environment. Contaminant components of its natural environment are materials that would typically interfere with diagnostic or therapeutic uses for the oligopeptide, and may include enzymes, hormones, and other proteinaceous or non-proteinaceous solutes. In preferred embodiments, the oligopeptide will be purified (1) to a degree sufficient to obtain at least 15 residues of N-terminal or internal amino acid sequence by use of a spinning cup sequenator, or (2) to homogeneity by SDS-PAGE under nonreducing or reducing conditions using Coomassie blue or, preferably, silver stain. Ordinarily, however, isolated polypeptide will be prepared by at least one purification step.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "epitope tagged" when used herein refers to a chimeric oligopeptide comprising a BDB oligopeptide fused to a "tag polypeptide". The tag polypeptide has enough residues to provide an epitope against which an antibody can be made, yet is short enough such that it does not interfere with activity of the oligopeptide to which it is fused. The tag polypeptide preferably also is fairly unique so that the antibody does not substantially cross-react with other epitopes. Optionally, the tag polypeptide is a carrier polypeptide that allows entry of the chimeric BDB oligopeptide into cells or is a proteinaceous toxin capable of inhibiting cell growth. Suitable tag polypeptides generally have at least six amino acid residues and usually between about 8 and 50 amino acid residues (preferably, between about 10 and 20 amino acid residues).

"IAP inhibitors" are molecules that block the anti-apoptotic activity of Inhibitor of Apoptosis Proteins (IAP). Examples of naturally occuring IAP inhibitors are the SMAC/DIABLO proteins in mammals, and the HID, RPR and GRIM proteins in Drosophila.

"Active" or "activity" for the purposes herein refers to form(s) of a BDB oligopeptide or BDB small organic molecule which retain a biological activity of native or naturally-occurring IAP inhibitors, wherein "biological" activity refers to a biological function caused by a native or naturally-occurring IAP inhibitor.

"IAPs" or Inhibitors of Apoptosis Proteins are molecules that inhibit apoptosis of a cell by physically interacting with, and blocking the action of, caspase molecules within the apoptosis pathway. Examples of IAP molecules are ML-IAP (Accession Number: BIR7_HUMAN), XIAP, NAIP, C-IAP1 and C-IAP2. Structurally, IAPs contain one or more BIR domains and most contain a carboxy terminal RING finger domain.

"Caspase" is defined as a cysteine protease polypeptide that cleaves polypeptide substrates on the C-terminal side of Aspartic acid residues.

"BIR domain containing polypeptide" is a polypeptide that contains an protein structure that is capable of specifically binding to and inhibiting, a caspase. Specifically, the BIR domain of ML-IAP is defined by amino acids 87-168 of the sequence disclosed in Accession Number: BIR7_HUMAN.

The term "antagonist" is used in the broadest sense, and includes any molecule that partially or fully blocks, inhibits, or neutralizes a biological activity of an IAP polypeptide. In a similar manner, the term "agents" is used in the broadest sense and includes any molecule that mimics a biological activity of a native IAP polypeptide inhibitor. Suitable agents or antagonist molecules specifically include BDB oligopeptides, BDB small organic molecules. Methods for identifying antagonists of an IAP polypeptide may comprise contacting an IAP polypeptide with a candidate antagonist molecule and measuring a detectable change in one or more biological activities normally associated with the IAP polypeptide.

"Oligopeptides" are short amino acid sequences between 3 and 30 amino acid residues in length and encompass naturally occurring amino acid residues and non-naturally occurring analogs of residues which may be used singly or in combination with naturally occurring amino acid residues in order to give the oligopeptide a particular conformational specificity or a particular biological activity, such as resistance to proteolysis.

"Treating" or "treatment" or "alleviation" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for an IAP polypeptide-expressing cancer if, after receiving a therapeutic amount of a BDB oligopeptide or BDB small organic molecule according to the methods of the present invention, the patient shows observable and/or measurable reduction in or absence of one or more of the following: reduction in the number of cancer cells or absence of the cancer cells; reduction in the tumor size; inhibition (i.e., slow to some extent and preferably stop) of cancer cell infiltration into peripheral organs including the spread of cancer into soft tissue and bone; inhibition (i.e., slow to some extent and preferably stop) of tumor metastasis; inhibition, to some extent, of tumor growth; and/or relief to some extent, one or more of the symptoms associated with the specific cancer; reduced morbidity and mortality, and improvement in quality of life issues. To the extent the BDB oligopeptide or BDB small organic molecule may prevent growth and/or kill existing cancer cells, it may be cytostatic and/or cytotoxic. Reduction of these signs or symptoms may also be felt by the patient.

The above parameters for assessing successful treatment and improvement in the disease are readily measurable by routine procedures familiar to a physician. For cancer therapy, efficacy can be measured, for example, by assessing the time to disease progression (TTP) and/or determining the response rate (RR). Metastasis can be determined by staging tests and by bone scan and tests for calcium level and other enzymes to determine spread to the bone. CT scans can also be done to look for spread to the pelvis and lymph nodes in the area. Chest X-rays and measurement of liver enzyme levels by known methods are used to look for metastasis to the lungs and liver, respectively. Other routine methods for monitoring the disease include transrectal ultrasonography (TRUS) and transrectal needle biopsy (TRNB).

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of the treatment of, alleviating the symptoms of or diagnosis of a cancer refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN®, polyethylene glycol (PEG), and PLURONICS®.

By "solid phase" or "solid support" is meant a non-aqueous matrix to which a BDB oligopeptide or BDB small organic molecule of the present invention can adhere or attach. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a BDB oligopeptide) to a mammal. The components of the liposome are commonly arranged in a bilayer formation, similar to the lipid arrangement of biological membranes.

A "small" molecule or "small" organic molecule is defined herein to have a molecular weight below about 500 Daltons.

An "effective amount" of a BDB oligopeptide as disclosed herein is an amount sufficient to carry out a specifically stated purpose. An "effective amount" may be determined empirically and in a routine manner, in relation to the stated purpose.

The term "therapeutically effective amount" refers to an amount of a BDB oligopeptide effective to "treat" a disease or disorder in a subject or mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See the definition herein of "treating". To the extent the drug may prevent growth and/or kill existing cancer cells, it may be cytostatic and/or cytotoxic.

A "growth inhibitory amount" of a BDB oligopeptide is an amount capable of inhibiting the growth of a cell, especially tumor, e.g., cancer cell, either *in vitro* or *in vivo*. A "growth inhibitory amount" of a BDB oligopeptide for purposes of inhibiting neoplastic cell growth may be determined empirically and in a routine manner.

A "cytotoxic amount" of BDB oligopeptide is an amount capable of causing the destruction of a cell, especially tumor, e.g., cancer cell, either *in vitro* or *in vivo.* A "cytotoxic amount" of BDB binding oligopeptide for purposes of inhibiting neoplastic cell growth may be determined empirically and in a routine manner.

BDB oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

A BDB oligopeptide "which binds" an IAP polypeptide of interest, e.g. a tumor-associated polypeptide target, is one that binds the BIR domain with sufficient affinity such that the BDB oligopeptide is useful as a diagnostic and/or therapeutic agent in targeting a cell or tissue expressing the IAP polypeptide, and does not significantly cross-react with other proteins. In such embodiments, the extent of binding of the BDB oligopeptide to a "non-target" protein will be less than about 10% of the binding of the BDB oligopeptide to its particular target protein as determined by fluorescence polarization, fluorescence activated cell sorting (FACS) analysis or radioimmunoprecipitation (RIA). With regard to the binding of an BDB oligopeptide to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about $10^{-4}$ M, alternatively at least about $10^{-5}$ M, alternatively at least about $10^{-6}$ M, alternatively at least about $10^{-7}$ M, alternatively at least about $10^{-8}$ M, alternatively at least about $10^{-9}$ M, alternatively at least about $10^{-10}$ M, alternatively at least about $10^{-11}$ M, alternatively at least about $10^{-12}$ M, or greater. In one embodiment, the term "specific binding" refers to binding where a molecule binds to a particular polypeptide or epitope on a particular polypeptide without substantially binding to any other polypeptide or polypeptide epitope.

A BDB oligopeptide that "inhibits the growth of tumor cells expressing an IAP polypeptide" or a "growth inhibitory" BDB oligopeptide is one which results in measurable growth inhibition of cancer cells expressing or overexpressing the appropriate IAP polypeptide. Preferred growth inhibitory BDB, oligopeptides inhibit growth of BIR domain containing expressing tumor cells by greater than 20%, preferably from about 20% to about 50%, and even more preferably, by greater than 50% (e.g., from about 50% to about 100%) as compared to the appropriate control, the control typically being tumor cells not treated with the BDB oligopeptide being tested. Growth inhibition of tumor cells *in vivo* can be determined in various ways such as is described in the Experimental Examples section below.

A BDB oligopeptide which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses an IAP polypeptide.

Preferably the cell is a tumor cell, e.g., a prostate, breast, ovarian, stomach, endometrial, lung, kidney, colon, melanoma or bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the BDB oligopeptide which induces apoptosis is one which results in about 2 to 50 fold, preferably about 5 to 50 fold, and most preferably about 10 to 50 fold, induction of annexin binding relative to untreated cell in an annexin binding assay.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, as well as head and neck cancer, and associated metastases.

The terms "cell proliferative disorder" and "proliferative disorder" refer to disorders that are associated with some degree of abnormal cell proliferation. In one embodiment, the cell proliferative disorder is cancer.

"Tumor", as used herein, refers to all neoplastic cell growth and proliferation, whether malignant or benign, and all pre-cancerous and cancerous cells and tissues.

A "BIR domain containing-expressing cell" is a cell which expresses an endogenous or transfected IAP polypeptide. An "IAP polypeptide-expressing cancer" is a cancer comprising cells that have an IAP polypeptide present. An "IAP polypeptide-expressing cancer" optionally produces sufficient levels of IAP polypeptide in cells thereof, such that a BDB oligopeptide can bind thereto and have a therapeutic effect with respect to the cancer. In another embodiment, a "BIR domain containing-expressing cancer" optionally produces sufficient levels of IAP polypeptide, such that a BDB oligopeptide can bind thereto and have a therapeutic effect with respect to the cancer. A cancer which "overexpresses" an IAP polypeptide is one which has significantly higher levels of IAP polypeptide in the cell, compared to a noncancerous cell of the same tissue type. Such overexpression may be caused by gene amplification or by increased transcription or translation. IAP polypeptide overexpression may be determined in a diagnostic or prognostic assay by evaluating increased levels of the IAP protein present in a cell, (e.g., via an immunohistochemistry assay using anti-IAP polypeptide antibodies prepared against an isolated IAP polypeptide which may be prepared using recombinant DNA technology from an isolated nucleic acid encoding the IAP polypeptide; FACS analysis, etc.). Alternatively, or additionally, one may measure levels of IAP polypeptide-encoding nucleic acid or mRNA in the cell, e.g., via fluorescent *in situ* hybridization using a nucleic acid based probe corresponding to an IAP-encoding nucleic acid or the complement thereof; (FISH; see WO98/4547 published October, 1998), Southern blotting, Northern blotting, or polymerase chain reaction (PCR) techniques, such as real time quantitative PCR (RT-PCR). One may also study IAP polypeptide overexpression by measuring shed antigen in a biological fluid such as serum, e.g, using antibody-based assays (see also, e.g., U.S. Patent No. 4,933,294 issued June 12, 1990; WO91/05264 published April 18, 1991; U.S. Patent 5,401,638 issued March 28, 1995; and Sias et al., J. Immunol. Methods 132:73-80 (1990)). Aside from the above assays, various *in vivo* assays are available to the skilled practitioner. For example, one may expose cells within the body of the patient to an antibody which is optionally labeled with a detectable label, e.g., a radioactive isotope, and binding of the antibody to cells in the patient can be evaluated, e.g., by external scanning for radioactivity or by analyzing a biopsy taken from a patient previously exposed to the antibody.

The word "label" when used herein refers to a detectable compound or composition which is conjugated directly or indirectly to the BDB oligopeptide so as to generate a "labeled" BDB oligopeptide. The label may be detectable by itself (e.g. radioisotope labels or fluorescent labels) or, in the case of an enzymatic label, may catalyze chemical alteration of a substrate compound or composition which is detectable.

The term "cytotoxic agent" as used herein refers to a substance that inhibits or prevents the function of cells and/or causes destruction of cells. The term is intended to include radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic agents e.g. methotrexate, adriamycin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "growth inhibitory agent" when used herein refers to a compound or composition which inhibits growth of a cell, especially an IAP-expressing cancer cell, either *in vitro* or *in vivo*. Thus, the growth inhibitory agent may be one which significantly reduces the percentage of IAP-expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-a-L-lyxo-hexapyranosyl)oxy]-7,8,9,10-tetrahydro-6,8,11-trihydroxy-8-(hydroxyacetyl)-1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon -$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL- 1a, IL-2, IL-3, IL-4, IL-5, IL-6, IL-7, IL-8, IL-9, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

The term "package insert" is used to refer to instructions customarily included in commercial packages of therapeutic products, that contain information about the indications, usage, dosage, administration, contraindications and/or warnings concerning the use of such therapeutic products.

## Compositions and Methods of the Invention

## Maytansine and maytansinoids

In one preferred embodiment, a BDB oligopeptide of the invention is conjugated to one or more maytansinoid molecules.

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

## Calicheamicin

Another conjugate of interest comprises a BDB oligopeptide conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, $\gamma_1^I$, $\alpha_2^I$, $\alpha_3^I$, N-acetyl-$\gamma_1^I$, PSAG and $\theta_1^I$ (Hinman et al., Cancer Research 53:3336-3342 (1993), Lode et al., Cancer Research 58:2925-2928 (1998) and the aforementioned U.S. patents to American Cyanamid). Another anti-tumor drug that the BDB oligopeptide can be conjugated to is QFA which is an antifolate. Both calicheamicin and QFA have intracellular sites of action and do not readily cross the plasma membrane. Therefore, cellular uptake of these agents through internalization processes greatly enhances their cytotoxic effects.

Other cytotoxic agents

Other antitumor agents that can be conjugated to the BDB oligopeptides or BDB small organic molecules of the invention include; adriamycin (doxorubicin), 4-tertiary butylphenol etoposide, taxol, camptothecin, methotrexate, vincristine or tamoxifen, BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

For selective destruction of the tumor, the BDB oligopeptide may comprise a highly radioactive atom. A variety of radioactive isotopes are available for the production of radioconjugated proteins. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the conjugate is used for diagnosis, it may comprise a radioactive atom for scintigraphic studies, for example $Tc^{99m}$ or $I^{123}$, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the BDB oligopeptide may be biosynthesized or may be synthesized by chemical amino acid synthesis using suitable amino acid precursors involving, for example, fluorine-19 in place of hydrogen. Labels such as $Tc^{99m}$ or $I^{123}$, $Re^{186}$, $Re^{188}$ and $In^{111}$ can be attached via a cysteine residue in the peptide. Yttrium-90 can be attached via a lysine residue. The IODOGEN method (Fraker et al., (1978) Biochem. Biophys. Res. Commun. 80: 49-57) can be used to incorporate iodine-123. "Monoclonal Antibodies in Immunoscintigraphy" (Chatal,CRC Press 1989) describes other methods in detail.

Conjugates of the BDB oligopeptide and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the BDB oligopeptide. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein comprising the BDB oligopeptide and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the BDB oligopeptide may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

A. BDB Oligopeptides

BDB oligopeptides of the present invention are oligopeptides that bind, preferably specifically, to an IAP polypeptide as described herein. BDB oligopeptides may be chemically synthesized using known oligopeptide synthesis methodology or may be prepared and purified using recombinant technology. BDB oligopeptides are usually at least about 3 amino acids in length, alternatively at least about 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a IAP polypeptide as described herein. BDB oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci.

U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large oligopeptide libraries to identify member(s) of those libraries which are capable of specifically binding to a polypeptide target. Phage display is a technique by which variant polypeptides are displayed as fusion proteins to the coat protein on the surface of bacteriophage particles (Scott, J.K. and Smith, G. P. (1990) Science 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natl. Acad. Sci. USA, 87:6378) or protein (Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren, Z-J. et al. (1998) Gene 215:439; Zhu, Z. (1997) CAN 33: 534; Jiang, J. et al. (1997) can 128:44380; Ren, Z-J. et al. (1997) CAN 127:215644; Ren, Z-J. (1996) Protein Sci. 5: 1833; Efimov, V. P. et al. (1995) Virus Genes 10:173) and T7 phage display systems (Smith, G. P. and Scott, J.K. (1993) Methods in Enzymology, 217, 228-257; U.S. 5,766,905) are also known.

Many other improvements and variations of the basic phage display concept have now been developed. These improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphylo-coccus aureus* protein A as an affinity tag has also been reported (Li et al., (1998) Mol Biotech. 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

BDB oligopeptides as described herein fused with another polypeptide sequence to generate a chimeric BDB oligopeptide are also contemplated. Research on Drosophila protein ANTENNAPEDIA, discovered that 16 amino acids of the 3rd helical domain could translocate across the cell membrane and enter into the cytoplasm intact (Prochiantz A., (1996) Curr. Opinion Neurobiol. (5):629-34, Derossi et al., (1998) Trends Cell Biol. (8) 84-87). This peptide was given the designation Penetratin (RQIKIWFQNRRMKWKK-NH2 (SEQ ID NO:54)). The mechanism by which the membrane translocation occurs is not yet defined, but recovery of the ANTENNAPEDIA peptide from the cytoplasm without degradation, and its low cell toxicity suggested that it could be fused to other peptide sequences to create chimeric molecules that could contact cells and be easily internalized with no loss of activity. The advantages of such a chimeric molecule are that no chemical coupling reaction is necessary, and the chimera can be either synthesized directly or inserted into a plasmid expression vector. Further advantages are that the Penetratin sequence can be fused with modified, for example biotinylated or phosphorylated, oligopeptides. An epitope tag sequence can be further added to facilitate recovery of the chimeric molecule with an antibody. Penetratin/SMAC fusions have been designed and shown to successfully interact with IAPs (Arnt et al., (2002) Jour. Bio. Chem. 277 (46) 44236-4424300). Arnt et al., fused 4-8 amino acids of SMAC to the Penetratin sequence, and included a biotynlated version. After a 30 minute incubation, the SMAC/Penetratin fusion polypeptide was recovered by streptavidin-agarose, the bound molecules were separated by SDS-PAGE and analyzed by immunoblotting. This experiment showed that the SMAC/Penetratin fusion oligopeptide bound to XIAP and cIAP1 in both cell lines tested. This group further demonstrated that the SMAC/Penetratin fusion oligopeptide was active as it was an effective inhibitor of IAPs, as caspase activity was increased. While the Penetratin molecule is specifically described here, other oligopeptides that have been demonstrated to be internalized, such as TAT transcription factor, Herpes VP22, FGF-2 and lactoferrin are also contemplated.

B. Screening for BDB Oligopeptides With the Desired Properties

Techniques for generating oligopeptides that bind to IAP polypeptides have been described above.

The growth inhibitory effects of a BDB oligopeptide of the invention may be assessed by methods known in the art, e.g., using cells which express an IAP polypeptide either endogenously or following transfection with the IAP gene. For example, appropriate tumor cell lines and IAP-transfected cells may be treated with a BDB oligopeptide of the invention at various concentrations for a few days (e.g., 2-7) days and stained with crystal violet or MTT or analyzed by some other colorimetric assay. Another method of measuring proliferation would be by comparing $^3$H-thymidine uptake by the cells treated in the presence or absence a BDB oligopeptide of the invention. After treatment, the cells are harvested and the amount of radioactivity incorporated into the DNA quantitated in a scintillation counter. Appropriate positive controls include treatment of a selected cell line with a growth inhibitory antibody, oligopeptide or small organic molecule known to inhibit growth of that cell line. Growth inhibition of tumor cells *in vivo* can be determined in various ways known in the art. Preferably, the tumor cell is one that overexpresses an IAP polypeptide. Preferably, the BDB oligopeptide will cause apoptosis of a IAP-expressing tumor cell *in vitro* or *in vivo* by about 25-100% compared to the untreated tumor cell, more preferably, by about 30-100%, and even more preferably by about 50-100% or 70-100%.

To select for a BDB oligopeptide which induces cell death, loss of membrane integrity as indicated by, e.g., propidium iodide (PI), trypan blue or 7AAD uptake may be assessed relative to control. A PI uptake assay can be performed in the absence of complement and immune effector cells. IAP polypeptide-expressing tumor cells are incubated with medium alone or medium containing the appropriate BDB oligopeptide. The cells are incubated for a 3 day time period. Following each treatment, cells are washed and aliquoted into 35 mm strainer-capped 12 x 75 tubes (1ml per tube, 3 tubes per treatment group) for removal of cell clumps. Tubes then receive PI (10μg/ml). Samples may be analyzed using a FACSCAN® flow cytometer and FACSCONVERT® CellQuest software (Becton Dickinson). Those BDB oligopeptides that induce statistically significant levels of cell death as determined by PI uptake may be selected as cell death-inducing BDB oligopeptides.

To screen for BDB oligopeptides which bind to a BIR domain on a IAP polypeptide of interest, polarization instrumentation such as an Analyst™ HT 96-384 (Molecular Devices Corp.) can be used. Samples for fluorescence polarization affinity measurements may be prepared in polarization buffer such as 50 mM Tris [pH 7.2], 120 mM NaCl, 1% bovine globulins and 0.05% octylglucoside) with 5-carboxyflourescein-conjugated peptides at 3-5 nM final concentrations. After an incubation step the reactions can be determined with standard cut-off filters for the fluorescein fluorophore (lex = 485 nm; lem = 530 nm) in 96-well black HE96 plates (Molecular Devices Corp.). The apparent Kd values can be determined from the EC50 values. The inhibition constants (Ki) can be determined as described previously (Keating et al., (2000) Proceedings of SPIE: In vitro diagnostic instrumentation Cohn, G.E., Ed. p128-137).

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val;met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |

(continued)

| Original Residue | Exemplary Substitutions | Preferred Substitutions |
|---|---|---|
| Tyr (Y) | trp; phe; thr; ser | phe |
| Val (V) | ile; leu; met; phe; ala; norleucine | leu |

Substantial modifications in function of BDB oligopeptide are accomplished by selecting substitutions that differ significantly in their effect on maintaining (a) the structure of the BDB oligopeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the BDB oligopeptide variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244:1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isosteric amino acid can be used.

Any cysteine residue not involved in maintaining the proper conformation of the BDB oligopeptide also may be substituted, generally with serine, to improve the oxidative stability of the molecule and prevent aberrant crosslinking. Conversely, cysteine bond(s) may be added to the BDB oligopeptide to improve its stability

A particularly preferred type of substitutional variant involves substituting one or more residues of a parent BDB oligopeptide. Generally, the resulting variant(s) selected for further development will have improved biological properties relative to the parent BDB oligopeptide from which they are generated. A convenient way for generating such substitutional variants involves affinity maturation using phage display. Briefly, a BDB oligopeptide contact site is mutated to generate amino substitutions at the site. The BDB oligopeptide variants thus generated are displayed in a monovalent fashion from filamentous phage particles as fusions to the gene III product of M13 packaged within each particle. The phage-displayed variants are then screened for their biological activity (e.g., binding affinity) as herein disclosed. In order to identify candidate region sites, for modification, alanine scanning mutagenesis can be performed to identify hypervariable region residues contributing significantly to BDB oligopeptide binding. Additionally, it is beneficial to analyze a crystal structure of the BDB oligopeptide/BIR domain complex to identify contact points between the BDB oligopeptide and the IAP polypeptide BIR domain. Such contact residues and neighboring residues are candidates for substitution according to the techniques elaborated herein. Once such variants are generated, the panel of variants is subjected to screening as described herein and BDB oligopeptide with superior properties in one or more relevant assays may be selected for further development.

2. Selection and Transformation of Host Cells

Host cells are transfected or transformed with expression or cloning vectors described herein for IAP polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as P. *aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan^r; E. coli* W3110 strain 37D6, which has the complete genotype *tonA ptr3 phoA E15 (argF-lac)169 degP ompT rbs7 ilvG kan^r; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

Full length BDB oligopeptides and BDB oligopeptides fusion proteins can be produced in bacteria, in particular when glycosylation is not needed, such as when the therapeutic BDB oligopeptide is conjugated to a cytotoxic agent (e.g., a toxin) and the conjugate by itself shows effectiveness in tumor cell destruction. Production in E. coli is faster and more cost efficient. For expression of BDB oligopeptides in bacteria, see, e.g., U.S. 5,648,237 (Carter et. al.), U.S. 5,789,199 (Joly et al.), and U.S. 5,840,523 (Simmons et al.) which describes translation initiation regions (TIR) and signal sequences for optimizing expression and secretion, these patents incorporated herein by reference. After expression, the BDB oligopeptide is isolated from the E. coli cell paste in a soluble fraction and can be purified.

In addition to prokaryotes, eukaryotic microbes such as filamentous fungi or yeast are suitable cloning or expression hosts for BDB oligopeptide encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9: 968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated BDB oligopeptides are derived from multicellular organisms. Examples of invertebrate cells include insect cells such as Drosophila S2 and Spodoptera Sf9, as well as plant cells, such as cell cultures of cotton, corn, potato, soybean, petunia, tomato, and tobacco. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for BDB oligopeptide production and cultured in conventional nutrient media modified as appropriate for inducing promoters, selecting transformants, or amplifying the genes encoding the desired sequences.

3. Selection and Use of a Replicable Vector

The nucleic acid (e.g., cDNA or genomic DNA) encoding BDB oligopeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The BDB oligopeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the BDB oligopeptide-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the BDB oligopeptide-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the BDB oligopeptide-encoding nucleic acid sequence to direct mRNA synthesis. Promoters recognized by a variety of potential host cells are well known. Promoters suitable for use with prokaryotic hosts include the β-lactamase and lactose promoter systems [Chang et al., (1978) Nature, 275:615; Goeddel et al., (1979) Nature, 281:544], alkaline phosphatase, a tryptophan (trp) promoter system [Goeddel, (1980)Nucleic Acids Res., 8:4057; EP 36, 776], and hybrid promoters such as the tac promoter [deBoer et al., (1983) Proc. Natl. Acad. Sci. USA, 80:21-25]. Promoters for use in bacterial systems also will contain a Shine-Dalgarno (S.D.) sequence operably linked to the DNA encoding BDB oligopeptides.

Examples of suitable promoting sequences for use with yeast hosts include the promoters for 3-phosphoglycerate kinase [Hitzeman et al., (1980) J. Biol, Chem., 255:2073] or other glycolytic enzymes [Hess et al., (1968) J. Adv. Enzyme Reg., 7:149; Holland, (1978) Biochemistry, 17:4900], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast Promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

BDB oligopeptide transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the BDB oligopeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the BDB oligopeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding BDB oligopeptides.

Still other methods, vectors, and host cells suitable for adaptation to the synthesis of BDB oligopeptide in recombinant vertebrate cell culture are described in Gething et al., Nature, 293:620-625 (1981); Mantei et al., Nature, 281:40-46 (1979); EP 117,060; and EP 117,058.

4. Culturing the Host Cells

The host cells used to produce the BDB oligopeptides of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem.102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### C. Diagnosis and Treatment with BDB Oligopeptides

To determine IAP expression in the cancer, various diagnostic assays are available. In one embodiment, IAP polypeptide overexpression may be analyzed by immunohistochemistry (IHC). Paraffin embedded tissue sections from a tumor biopsy may be subjected to the IHC assay and accorded an IAP protein staining intensity criteria as follows:

Score 0 - no staining is observed or is observed in less than 10% of tumor cells.
Score 1+ - a faint/barely perceptible staining is detected in more than 10% of the tumor cells.
Score 2+ - a weak to moderate staining is observed in more than 10% of the tumor cells.
Score 3+ - a moderate to strong staining is observed in more than 10% of the tumor cells.

Those tumors with 0 or 1+ scores for IAP polypeptide expression may be characterized as not overexpressing IAP, whereas those tumors with 2+ or 3+ scores may be characterized as overexpressing IAP.

Alternatively, or additionally, FISH assays such as the INFORM® (sold by Ventana, Arizona) or PATHVISION® (Vysis, Illinois) may be carried out on formalin-fixed, paraffin-embedded tumor tissue to determine the extent (if any) of IAP overexpression in the tumor.

IAP overexpression or amplification may be evaluated using an *in vivo* diagnostic assay, e.g., by administering a molecule (such as an antibody, oligopeptide or organic molecule) which binds the molecule to be detected and is tagged with a detectable label (e.g., a radioactive isotope or a fluorescent label) and externally scanning the patient for localization of the label.

As described above, the BDB oligopeptides of the invention have various non-therapeutic applications. The BDB oligopeptides of the present invention can be useful for diagnosis and staging of IAP polypeptide-expressing cancers (e.g., in radioimaging). The BDB oligopeptides are also useful for detection and quantitation of IAP polypeptide *in vitro,* e.g., to kill and eliminate IAP-expressing cells from a population of mixed cells as a step in the purification of other cells.

Currently, depending on the stage of the cancer, cancer treatment involves one or a combination of the following therapies: surgery to remove the cancerous tissue, radiation therapy, and chemotherapy. BDB oligopeptide therapy may be especially desirable in elderly patients who do not tolerate the toxicity and side effects of chemotherapy well and in metastatic disease where radiation therapy has limited usefulness. The tumor targeting BDB oligopeptides of the invention are useful to alleviate IAP-expressing cancers upon initial diagnosis of the disease or during relapse. For therapeutic applications, the BDB oligopeptide can be used alone, or in combination therapy with, e.g., hormones, antiangiogens, or radiolabelled compounds, or with surgery, cryotherapy, and/or radiotherapy. BDB oligopeptide treatment can be administered in conjunction with other forms of conventional therapy, either consecutively with, pre- or post-conventional therapy. Chemotherapeutic drugs such as TAXOTERE® (docetaxel), TAXOL® (paclitaxel), estramustine and mitoxantrone are used in treating cancer, in particular, in good risk patients. In the present method of the invention for treating or alleviating cancer, the cancer patient can be administered BDB oligopeptide in conjunction with treatment with the one or more of the preceding chemotherapeutic agents. In particular, combination therapy with paclitaxel and modified derivatives (see, e.g., EP0600517) is contemplated. The BDB oligopeptide will be administered with a therapeutically effective dose of the chemotherapeutic agent. In another embodiment, the BDB oligopeptide is administered in conjunction with chemotherapy to enhance the activity and efficacy of the chemotherapeutic agent, e.g., paclitaxel. The Physicians' Desk Reference (PDR) discloses dosages of these agents that have been used in treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician.

In one particular embodiment, a conjugate comprising an BDB oligopeptide conjugated with a cytotoxic agent is administered to the patient. In a preferred embodiment, the cytotoxic agent targets or interferes with the nucleic acid in the cancer cell. Examples of such cytotoxic agents are described above and include maytansinoids, calicheamicins, ribonucleases and DNA endonucleases.

The BDB oligopeptides or toxin conjugates thereof are administered to a human patient, in accord with known methods, such as intravenous administration, e.g., as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the BDB oligopeptide is preferred.

Other therapeutic regimens may be combined with the administration of the BDB oligopeptide. The combined administration includes co-administration, using separate formulations or a single pharmaceutical formulation, and consecutive administration in either order, wherein preferably there is a time period while both (or all) active agents simultaneously exert their biological activities. Preferably such combined therapy results in a synergistic therapeutic effect. As an example, administration of a BDB oligopeptide in combination with the pro-apoptotic polypeptide APO2L/TRAIL (Accession Number: TN10_HUMAN). In a prostate tumor cell line, it was shown that tranfection of the tumor cells with a SMAC cDNA could sensitize the tumor cells to APO2L/TRAIL, and led to a reduction of XIAP, c-IAP1 and c-IAP2 (Ng et al., (2002) Mol. Cancer Ther. (12) 1051-1058). Further experiments showed that overexpression of SMAC in Jurkat cells sensitized them significantly to APO2L/TRAIL induced apoptosis (Guo et al., (2002) Blood 99, 3419-3426). In a different set of experiments, U87MG glioma cells were implanted into the striatum of athymic mice, allowed to establish a tumor, and then a combination of SMAC peptide and APO2L/TRAIL was administered. While administration of APO2L/TRAIL alone resulted in reduction in size of the tumor, a combination of SMAC peptide and APO2L/TRAIL resulted in complete eradication (Fulda et al., (2002) Nature Medicine (8) 808-815). Mice treated with the SMAC peptide and APO2L/TRAIL combination also survived significantly longer than the mice treated with APO2L/TRAIL alone or with controls.

It may also be desirable to combine administration of the BDB oligopeptides, with administration of an antibody directed against another tumor antigen associated with the particular cancer.

In another embodiment, the therapeutic treatment methods of the present invention involves the combined administration of BDB oligopeptides and one or more chemotherapeutic agents or growth inhibitory agents, including co-administration of cocktails of different chemotherapeutic agents. Chemotherapeutic agents include estramustine phosphate, prednimustine, cisplatin, 5-fluorouracil, melphalan, cyclophosphamide, hydroxyurea and hydroxyureataxanes (such as paclitaxel and doxetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

The BDB oligopeptide may be combined with an anti-hormonal compound; e.g., an anti-estrogen compound such as tamoxifen; an anti-progesterone such as onapristone (see, EP 616 812); or an anti-androgen such as flutamide, in dosages known for such molecules. Where the cancer to be treated is androgen independent cancer, the patient may previously have been subjected to anti-androgen therapy and, after the cancer becomes androgen independent, the BDB oligopeptide (and optionally other agents as described herein) may be administered to the patient.

Sometimes, it may be beneficial to also co-administer a cardioprotectant (to prevent or reduce myocardial dysfunction associated with the therapy) or one or more cytokines to the patient. In addition to the above therapeutic regimes, the patient may be subjected to surgical removal of cancer cells and/or radiation therapy, before, simultaneously with, or post antibody, oligopeptide or organic molecule therapy. Suitable dosages for any of the above co-administered agents are those presently used and may be lowered due to the combined action (synergy) of the agent and BDB oligopeptide.

For the prevention or treatment of disease, the dosage and mode of administration will be chosen by the physician according to known criteria. The appropriate dosage of BDB oligopeptide will depend on the type of disease to be treated, as defined above, the severity and course of the disease, whether the BDB oligopeptide is administered for preventive or therapeutic purposes, previous therapy, the patient's clinical history and response to the BDB oligopeptide, and the discretion of the attending physician. The BDB oligopeptide is suitably administered to the patient at one time or over a series of treatments. Preferably, the BDB oligopeptide is administered by intravenous infusion or by subcutaneous injections. Aside from administration of the BDB oligopeptide to the patient, the present application contemplates administration of the BDB oligopeptide by gene therapy. Such administration of nucleic acid encoding the BDB oligopeptide is encompassed by the expression "administering a therapeutically effective amount of an BDB oligopeptide". See, for example, WO96/07321 published March 14, 1996 concerning the use of gene therapy to generate intracellular antibodies.

There are two major approaches to getting the nucleic acid (optionally contained in a vector) into the patient's cells; *in vivo* and *ex vivo*. For *in vivo* delivery the nucleic acid is injected directly into the patient, usually at the site where the BDB oligopeptide is required. For *ex vivo* treatment, the patient's cells are removed, the nucleic acid is introduced into these isolated cells and the modified cells are administered to the patient either directly or, for example, encapsulated within porous membranes which are implanted into the patient (see, e.g., U.S. Patent Nos. 4,892,538 and 5,283,187). There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for *ex vivo* delivery of the gene is a retroviral vector.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of the currently known gene marking and gene therapy protocols see Anderson et al., Science 256:808-813 (1992). See also WO 93/25673 and the references cited therein.

The present BDB oligopeptide are useful for treating a IAP-expressing cancer or alleviating one or more, symptoms of the cancer in a mammal. Such a cancer includes melanoma, prostate cancer, cancer of the urinary tract, lung cancer, breast cancer, colon cancer and ovarian cancer, more specifically, prostate adenocarcinoma, renal cell carcinomas, colorectal adenocarcinomas, lung adenocarcinomas, lung squamous cell carcinomas, and pleural mesothelioma. The cancers encompass metastatic cancers of any of the preceding. The BDB oligopeptide is able to bind to at least a portion of the cancer cells that express IAP polypeptide in the mammal. In a preferred embodiment, the BDB oligopeptide is effective to destroy or kill IAP-expressing tumor cells or inhibit the growth of such tumor cells, *in vitro* or *in vivo,* upon binding to IAP polypeptide on the cell.

The invention provides a composition comprising a BDB oligopeptide of the invention, and a carrier. For the purposes of treating cancer, compositions can be administered to the patient in need of such treatment, wherein the composition can comprise one or more BDB oligopeptides. In a further embodiment, the compositions can comprise these BDB oligopeptides in combination with other therapeutic agents such as cytotoxic or growth inhibitory agents, including chemotherapeutic agents. The invention also provides formulations comprising BDB oligopeptide of the invention, and a carrier. In one embodiment, the formulation is a therapeutic formulation comprising a pharmaceutically acceptable carrier.

The invention also provides methods useful for treating a IAP polypeptide-expressing cancer or alleviating one or more symptoms of the cancer in a mammal, comprising administering a therapeutically effective amount of an BDB oligopeptide to the mammal. The BDB oligopeptide therapeutic compositions can be administered short term (acute) or chronic, or intermittent as directed by physician. Also provided are methods of inhibiting the growth of, and killing an IAP polypeptide-expressing cell.

The invention also provides kits and articles of manufacture comprising at least one BDB oligopeptide. Kits containing BDB oligopeptides find use, e.g., for cell killing assays. For example, for isolation and purification of IAP, the kit can contain a BDB oligopeptide coupled to beads (e.g., sepharose beads). Kits can be provided which contain the BDB oligopeptides for detection and quantitation of IAP *in vitro.* Such BDB oligopeptide useful for detection may be provided with a label such as a fluorescent or radiolabel.

E. Articles of Manufacture and Kits

Another embodiment of the invention is an article of manufacture containing materials useful for the treatment of IAP expressing cancer. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the cancer condition and may have a sterile access port (for example the container may be an intravenous solution bag or a vial having a stopper pierceable by a hypodermic injection needle). At least one active agent in the composition is a BDB oligopeptide of the invention. The label or package insert indicates that the composition is used for treating cancer. The label or package insert will further comprise instructions for administering the BDB oligopeptide composition to the cancer patient. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes, e.g., for IAP-expressing cell killing assays or for purification of IAP polypeptide from cells. For isolation and purification of IAP polypeptide, the kit can contain an BDB oligopeptide coupled to beads (e.g., sepharose beads). Kits can be provided which contain the BDB oligopeptides for detection and quantitation of IAP polypeptide *in vitro*. As with the article of manufacture, the kit comprises a container and a label or package insert on or associated with the container. The container holds a composition comprising at least one BDB oligopeptide of the invention. Additional containers may be included that contain, e.g., diluents and buffers, control antibodies, oligopeptides or small organic molecules. The label or package insert may provide a description of the composition as well as instructions for the intended in vitro or diagnostic use.

This invention encompasses methods of screening compounds to identify those that prevent the effect of the IAP polypeptide (antagonists). Screening assays for antagonist drug candidates are designed to identify compounds that bind or complex with the IAP polypeptides. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with an IAP polypeptide under conditions and for a time sufficient to allow these two components to interact.

In binding assays, the interaction is binding and the complex formed can be isolated or detected in the reaction mixture. In a particular embodiment, the IAP polypeptide or the drug candidate is immobilized on a solid phase, e.g., on a microtiter plate, by covalent or non-covalent attachments. Non-covalent attachment generally is accomplished by coating the solid surface with a solution of the IAP polypeptide and drying. Alternatively, an immobilized antibody, e.g., a monoclonal antibody, specific for the IAP polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

To assay for antagonists, the IAP polypeptide may be added to a cell along with the compound to be screened for a particular activity and the ability of the compound to inhibit the activity of interest in the presence of the IAP polypeptide indicates that the compound is an antagonist to the IAP polypeptide. Alternatively, antagonists may be detected by combining the IAP polypeptide and a potential antagonist with IAP polypeptide under appropriate conditions for a competitive inhibition assay. The IAP polypeptide can be labeled, such as by radioactivity, such that the number of IAP polypeptide molecules bound to the competitor can be used to determine the effectiveness of the potential antagonist.

Potential antagonists include BDB oligopeptides that bind to the BIR domain of the IAP polypeptide, thereby blocking the normal biological activity of the IAP polypeptide. Such peptides can be synthesized chemically and/or produced by recombinant DNA technology. See, *e.g.*, Marasco et al., Proc. Natl. Acad. Sci. USA, 90: 7889-7893 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The following examples are offered for illustrative purposes only, and are not intended to limit the scope of the present invention in any way.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

EXAMPLE 1: Protein Expression and Purification.

Sequences encoding ML-IAP BIR (amino acid residues 63-179), X-IAP BIR2 (amino acid residues 124-240 with mutations C202A and C213G. (Sun et al., (1999) Nature 401, 818-822)), and X-IAP BIR3 (amino acids residues 241-356. Sun et al., (2000) J. Biol Chem. 275, 33777-33781 )) were subcloned into pET15b vectors (Novagen™) for bacterial expression. The vectors pET15b-XIAPBIR2C202AC218G, pET15b-XIAPBIR3, and pET15b-MLIAPBIR were introduced into Escherichia coli strain BL21 (DE3). Overnight cultures were diluted 1:100 and grown at 30°C in LB media with 50 mg/ml carbenicillin to an A600 of 0.5-0.7 with vigorous shaking. Isopropyl b-D-thiogalactoside (IPTG) was added to a final concentration of 1mM and cultures were grown for 4 hours at 30°C. One litre of frozen cell pellet was resuspended in 100 ml Buffer A (50 mM Tris [pH 8.0], 300mM NaCl, 5mM b-mercaptoethanol, 0.5mM PMSF, 2mM benzamidine) with 5mM imidazole and placed on ice for 30 minutes. Cells were lysed by homogenizing followed by repeated passes through a microfluidizer. Lysate was cleared by centrifugation at 15,000 rpm for 30 minutes. The supernatant was loaded onto a Ni-agarose column (Qiagen), washed with 10 column volumes Buffer A with 10 mM imidazole, and eluted with 10 column volumes Buffer A with 300 mM imidazole. Fractions containing BIR protein were pooled. Final concentrations of 5 mM DTT and 100mM zinc acetate were added. The pool was concentrated and loaded on a Superdex 75™ (Pharmacia) sizing column. Protein eluted over one column volume into 50 mM Tris 8.0, 300mM NaCl, 0.5 mM PMSF, 2 mM benzamidine, 5 mM DTT, 50 mM zinc acetate, 1 mM sodium azide. Fractions containing BIR protein were pooled and dialyzed against 4 changes of buffer containing 50 mM Tris (pH 8.0), 120 mM NaCl, 5 mM DTT, 0.5 mM PMSF, 2 mM benzamidine, 50 mM zinc acetate, 1 mM sodium azide. Protein was concentrated and stored at -80°C for further characterization.

EXAMPLE 2: Construction of Polyvalent Naïve Peptide-Phage Libraries.

Libraries were constructed using methods described previously (Sidhu et al., (2000) Methods Enzymology 328, 333-363), with a phagemid vector containing an IPTG-inducible Ptac promoter driving the expression of open reading frames encoding fusion proteins containing 6, 8, 14, or 20 residues, respectively.

EXAMPLE 3: Selection of BIR-Binding Phage.

Immunosorbant plates (Nunc Maxisorp™) were coated with 5 μg/ml of BIR domain (ML-IAP BIR, or XIAP-BIR2 or BIR3) in 50 mM sodium carbonate buffer (pH 9.6) for 1 hour at room temperature, followed by blocking for 1 hour with 0.2% BSA in phosphate-buffered saline (PBS). The plates were washed with PBS, 0.05% Tween 20. Phage from the four naïve peptide-phage libraries were pooled and cycled through three rounds of binding selection on immunosorbant plates coated with the BIR domains. In the first round, 4.8 ml of phage cocktail (~$10^{13}$ phage/ml in PBS, 0.05% Tween 20, 0.2% BSA (BSA/Tween buffer)) were added to 48 coated wells (100 μl/well). After 2 hours incubation at room temperature with shaking, the plate was washed with PBS, 0.05% Tween 20 to remove unbound phage. Bound phage were eluted with 0.2 M glycine, pH 2.0 (100 μl/well), and the phage eluant was neutralized by adding 1/6 volume of 1.0 M Tris, pH 8.0. The eluted phage were amplified overnight by propagation in E. coli XL1-blue™ cells (Stratagene) with M13-VCS™ helper phage (Stratagene) and harvested by precipitation with PEG/NaCl. The selection procedure for round three was identical to round one, while round two differed only in the use of 0.2% casein in place of BSA in both the blocking buffer and the phage cocktail. Individual phage clones from each round were analyzed for specific binding to BIR domains using single-point phage ELISAs (see Example 4). Positive clones, those that bound to the BIR domain but not BSA, were subjected to DNA sequence analysis.

EXAMPLE 4: Single-Point Phage ELISA.

Individual *E. coli* XL1-blue colonies harboring phagemids were picked into 500 μl of growth medium (2YT supplemented with 50 μg/ml carbenicillin and $10^{10}$ pfu/ml VCS-M13 helper phage) in 1.2-ml culture tubes in a 96-well format. After overnight growth, the tube racks were centrifuged for 10 minutes at 2,500 rpm. The phage supernatants (300 μl) were transferred to 96-well plates and used directly in single-point phage ELISAs. For phage ELISAs, 96-well Maxisorp™ immunoplates (NUNC) were coated with 100 μl of 5 μg/ml capture target protein blocked with BSA and washed (as described in Example 3). Phage supernatants (50 μl) were added to individual wells and incubated for 1 hour with shaking at room temperature. The plates were washed eight times with PBS, 0.05% Tween 20, incubated with 50 μl of 1:10,000 horse radish peroxidase/anti-M13 antibody conjugate (Pharmacia) in BSA/Tween buffer for 30 min, and then washed eight times with PBS, 0.05% Tween 20™ and two times with PBS. Plates were developed using a tetramethylbenzidine substrate (TMB, Kirkegaard and Perry, Gaithersburg, MD), quenched with 1.0 M $H_3PO^4$ (50 μl), and read spectrophotometrically at 450 nm.

EXAMPLE 5: Peptide Synthesis and Binding Assays.

*Peptide Synthesis*. Peptides were synthesized by either manual or automated (Milligen 9050™) solid-phase synthesis at 0.2 mM scale on PEG-polystyrene resin utilizing Fmoc chemistry. Purification was performed using HPLC with an $H_2O$-acetonitrile gradient with added 0.1% trifluroacetic acid. Masses of each peptide were verified by electrospray mass spectrometry.

To assay the synthesized peptides, polarization experiments were performed on an Analyst™ HT 96-384 (Molecular Devices Corp.). Samples for fluorescence polarization affinity measurements were prepared by addition of 1:2 serial dilutions of either ML-IAP-BIR, XIAP-BIR3 or XIAP-BIR2 in polarization buffer (50 mM Tris [pH 7.2], 120 mM NaCl, 1% bovine globulins and 0.05% octylglucoside) to 5-carboxyflourescein-conjugated peptides [AVPFAK(5-FAM)K (Hid-FAM) or AVPIAQKSEK(5-FAM) (SMAC-FAM)] at 3-5 nM final concentrations. The reactions were read after an incubation time of 10 minutes at room temperature with standard cut-off filters for the fluorescein fluorophore ($\lambda_{ex}$ = 485 nm; $\lambda_{em}$ = 530 nm) in 96-well black HE96™ plates (Molecular Devices Corp.). The apparent Kd values were determined from the EC50 values. Competition experiments were performed by addition of ML-IAP-BIR, XIAP-BIR3 or XIAP-BIR2 proteins at 1, 1-2, or 30 μM, respectively, for SMAC-FAM, or 0.2, 0.5, or 30 μM, respectively, for Hid-FAM, to wells containing probe as well as serial dilutions of the antagonists in the polarization buffer. Samples were read after a 10-minute incubation. The inhibition constants (Ki) for the peptides were determined as described previously (Keating et al., (2000) Proceedings of SPIE: In vitro diagnostic instrumentation Cohn, G.E., Ed. p128-137). The results of the peptide binding assays are shown in Table 1 below.

Table 1: $K_i$ values for phage-derived peptides

| Protein Target | Selected Phage-Peptide Sequence | $K_i$ (μM) | | |
|---|---|---|---|---|
| | | ML-IAP-BIR | XIAP-BIR3 | XIAP-BIR2 |
| | AVPIAQKSE (SEQ ID NO:1) | 0.5 | 0.67 | 13.8 |

(continued)

Table 1: $K_i$ values for phage-derived peptides

| Protein Target | Selected Phage-Peptide Sequence | $K_i$ (µM) | | |
|---|---|---|---|---|
| | | ML-IAP-BIR | XIAP-BIR3 | XIAP-BIR2 |
| ML-IAP BIR | AVPWGLKSE (SEQ ID NO:2) | 0.42 | 0.54 | 6.9 |
| ML-IAP BIR | AIPFEEKSE (SEQ ID NO:3) | 0.44 | 0.59 | 8.7 |
| ML-IAP BIR | AVPWIGKSE (SEQ ID NO:4) | 0.33 | 0.56 | 13.3 |
| XIAP-BIR3 | AVPFAVKSE (SEQ ID NO:5) | 0.16 | 0.39 | 14.7 |
| XIAP-BIR2 | AVGVPWKSE (SEQ ID NO:6) | 6.0 | >64 | 2.3 |
| XIAP-BIR2 | AEAVAWKSE (SEQ ID NO:7) | 9.3 | >64 | 2.5 |
| XIAP-BIR2 | ATAVIEKSE (SEQ ID NO:8) | 4.3 | >64 | 5.7 |
| XIAP-BIR2 | AEAVPWKSE (SEQ ID NO:9) | 2.1 | >64 | 3.6 |
| XIAP BIR2 | AEWAVKSE (SEQ ID NO:10) | 4.4 | >64 | 15 |
| XIAP BIR2 | AQAVAWKSE (SEQ ID NO:11) | 7 | >64 | 4.2 |

a SMAC-based control peptide. Other synthetic peptides correspond to six-residue phage-selected sequences appended with the KSE at their C-termini in order to ensure peptide solubility.

The peptides of SEQ ID NOs: 1 to 11 are listed for illustrative purposes only.

EXAMPLE 6: Crystallization and Data Collection.

Peptides were reconstituted from lyophilized powder in 10 mM MES, pH 6.5. Peptides were added to ML-IAP-BIR protein (20 mg/ml in its storage buffer) in a 2:1 molar excess of peptide. The peptide/protein complex was mixed in a 1:1 ratio with well solution (50 mM Na acetate, pH 5.0, 5%(v/v) PEG 300, 5mM DTT) and equilibrated by vapor diffusion against the well solution. An immediate precipitate formed upon mixing, which gave rise to an oily, foamy skin in one day followed by growth of small rod-shaped crystals over the next week. Crystals also grew using ethanol, isopropanol, or t-butanol as precipitants; PEG 300 was preferred since it could also act as a cryoprotectant.
Crystals were separated from the skin of the crystallization drop and transferred to a stabilizer containing 50 mM Na acetate, pH 5.0 and 10% (v/v) PEG 300, then transferred after 20 minutes to the same solution containing 20% PEG. After a further 20 minutes in the second cryostabilizer the crystals were frozen in liquid nitrogen. Data were collected and processed using HKL2000™ (or DENZO™ and SCALEPACK™ separately).

EXAMPLE 7: Structure Determination and Refinement.

MAD phasing, which takes advantage of the anomalous scattering of the naturally bound zinc in the protein, was used to solve the structure of the AEAVPWKSE (SEQ ID NO: 9) peptide complex. The zinc sites were clearly evident in anomalous and dispersive Patterson maps calculated from the MAD data, and five sites were found using the Patterson search routine in CNX™ (Accelrys, Inc.). Density modification including solvent flipping (using CNX™) produced easily interpretable density. The BIR domain (residues 217-310) of DIAP (PDB accesssion code 1JD4) was then manually placed into density for each of the five copies in the asymmetric unit, and rigid-body refined. The amino acid sequence of the model was changed to that of ML-IAP, and the resulting model was subjected to several rounds of simulated annealing, positional refinement, and individual atomic B-factor refinement using CNX™, interspersed with map inspection and model rebuilding (including building of the bound peptide). Both of the AVPIAQKSE (SEQ ID NO:1) and AEWAVKSE (SEQ ID NO: 10) complex structures were sufficiently isomorphous with the MAD-phased data (cross R factors below 15%) that inspection of Fo(new peptide)-Fo(MAD peak wavelength) maps, phased using the refined AEAVPWKSE (SEQ ID NO:9) complex structure, clearly indicated what changes were required to the model to account for the new peptide. These changes (primarily substituting the new amino acids in the bound peptide and adjustment of the conformation of Lys121) were made and the resulting model was put through one round of positional refinement and B-factor refinement.

EXAMPLE 8: Selection of IAP-Binding Peptides.

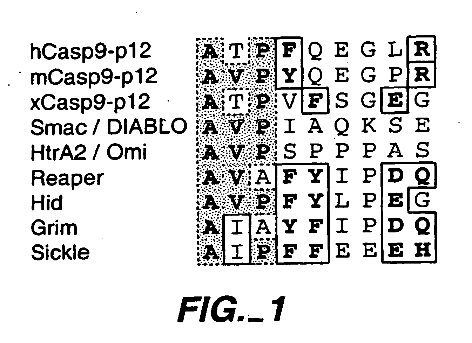

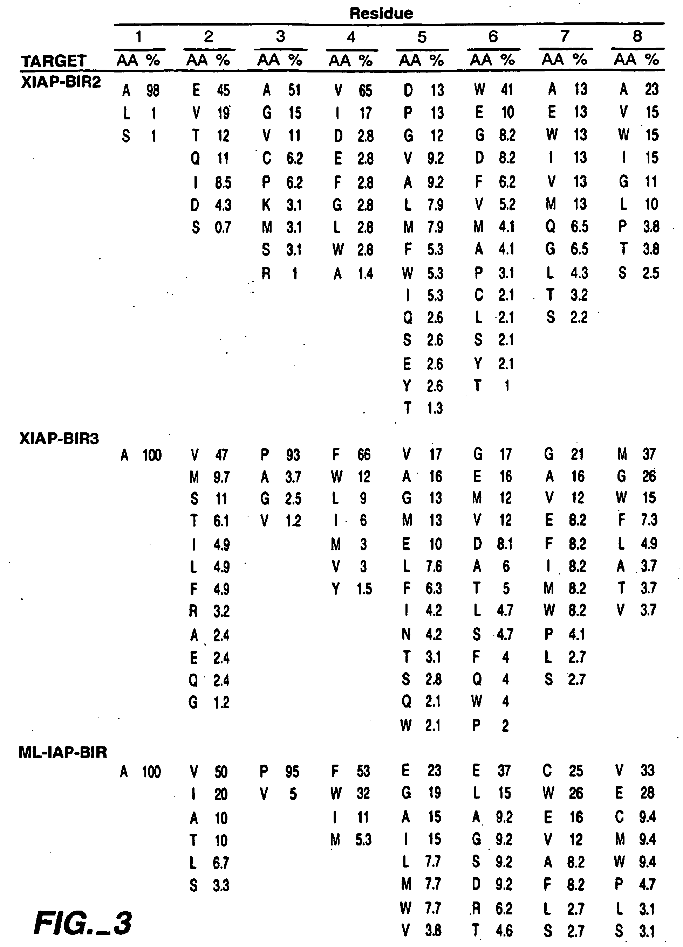

In order to ascertain the diversity of peptide sequences that bind to the BIR domains of ML-IAP and XIAP, multivalent M-13 phage libraries of linear peptides displayed on the gene-VIII phage coat protein were panned against ML-IAP-BIR, XIAP-BIR2, and XIAP-BIR3. Linear $X_6$, $X_8$, $X_{14}$, and $X_{20}$ libraries were prepared and combined to give a library with $8 \times 10^{10}$ members. A total of 96 individual clones were isolated for single point phage ELISA analysis after four rounds of sorting against each protein target. XIAP-BIR2 and -BIR3 yielded clones that demonstrated strong binding to the BIR domains with no detectable binding to BSA. sequencing revealed that 95% of the positive clones came from the $X_6$ and $X_8$ libraries, suggesting that the peptides were binding to localized epitopes in the protein domains.
Following four rounds of selection and amplification using the new combined $X_6$ and $X_8$ library ($4 \times 10^{10}$ members), clones were isolated that gave positive single point analysis for ML-IAP and no signal against BSA (Figure 3).
In these experiments the size of the libraries are greater than the theoretical diversity of both the $X_6$ and $X_8$ libraries. Thus, consensus sequences have been obtained from libraries that contain all possible amino acid combinations in the eight N-terminal positions. Similar sequences were obtained from sorts against both ML-IAP-BIR and XIAP-BIR3 (Figure 3). In both cases alanine is the only residue observed at the N-terminus. Approximately 50% of the clones have Val in position 2 and greater than 90% prefer Pro in position 3. The fourth position exhibits a strong selection bias for aromatic hydrophobic residues with phenylalanine the most common and tryptophan the second most common residue. No obvious bias is observed for any other position, suggesting that the four N-terminal residues are the most important for BIR binding. The consensus sequence for both ML-IAP-BIR and XIAP-BIR3, AVPF, corresponds with the N-terminus of Hid (Figure 1).
The results obtained from panning against XIAP-BIR2 differ significantly from those of ML-IAP-BIR and XIAP-BIR3. Although Alanine is again highly conserved at the N-terminus, the acidic amino acid Glutamic Acid is preferred at position 2. The small hydrophobic residues Glycine, Alanine, and Valine are selected at position 3 in contrast to the almost exclusive selection of proline in this position for ML-IAP-BIR and XIAP-BIR3. In position 4 the hydrophobic residues Valine and Isoleucine are favored over the larger aromatic amino acids found for ML-IAP-BIR and XIAP-BIR3.

EXAMPLE 9: Binding Affinities of Phage-Derived Peptides

In order to compare the relative affinities of these peptides for the different BIR domains, representative peptides were synthesized and affinities were determined using a fluorescence polarization-based competition assay with a SMAC-based 5-carboxyfluorescein-labeled peptide (SMAC-FAM) as the probe (Table 1). The affinities of the phage-selected peptides for their target proteins are, in general, slightly greater than for the SMAC-based peptide. The peptides selected for binding to XIAP-BIR2 exhibit > ~ 10-fold specificity for ML-IAP-BIR relative to XIAP-BIR3. These peptides are on the order of 10-fold weaker than the SMAC-based peptide for binding to ML-IAP-BIR, but > 100-fold weaker for binding to XIAP-BIR3.

EXAMPLE 10: Structure of ML-IAP BIR Bound to SMAC- and Phage-Derived Peptides

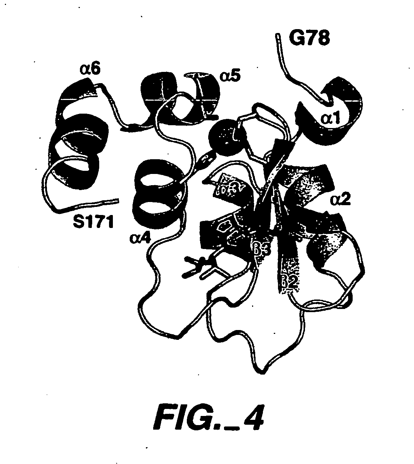

In order to further understand the specificity differences observed for some of the phage-derived peptides, we crystallized the BIR domain of ML-IAP in complex with the SMAC-based peptide (AVPIAQKSE (SEQ ID NO:1)) and with two different peptides of interest. AEAVPWKSE (SEQ ID NO:9) and AEVVAVKSE (SEQ ID NO:10). The structures were determined to resolutions between 2.2-2.8 Å (Table 2). In each case there are five copies of ML-IAP-BIR in the asymmetric unit of the crystals (Figure 2); the final atomic models contain protein residues 72-169 (protomer A), 72-171 (protomers B-D), and 78-171 (protomer E). Surprisingly, only one of the five BIR domains (protomer E) binds peptide in the SMAC-binding pocket; the bound peptides have strong electron density for residues 1'-4' and no density for the C-terminal five residues. Protomers A-D form a tetramer in which the SMAC-binding pocket is occupied by residues Ala73-Thr74-Leu75-Ser76 from a neighboring protomer. A heptaethylene glycol molecule further stabilizes this tetramer by interacting with a number of tyrosines at its center.
With the exception of the N-terminal region, which is disordered in the peptide-bound domain, and residues 116-119 which are found in two distinct conformations, the five BIR domains within each asymmetric unit are very similar with average backbone pairwise RMS deviations of ≤ 0.45 Å for superposition of residues 79-115 and 120-168. The BIR domain of ML-IAP comprises five α-helices, a three-stranded β-sheet, and a zinc atom chelated by three Cysteine and one Histidine residue (Figure 4). The structures are thus similar to those of other peptide-bound BIR domains, including XIAP-BIR3/SMAC (Wu et al., (2000) Nature 408, 1008-1012), XIAP-BIR2/caspase-3 (Riedl et al., (2001) Cell 104, 791-800), and DIAP1-BIR2/Grim and Hid (Wu et al., (2001) Mol. Cell 8, 95-104) complexes, with RMS deviations of < 0.7 Å for all aligned C-$\alpha$ atoms.

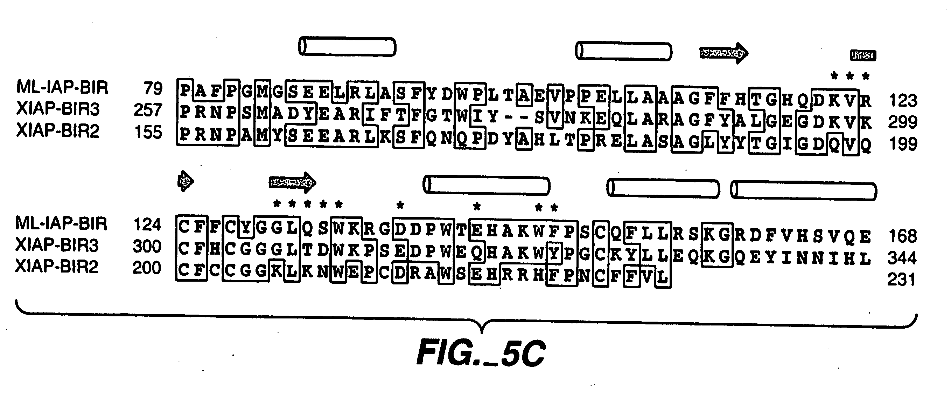

The binding interactions between ML-IAP-BIR and the SMAC- and phage-derived peptides are essentially identical to those observed for SMAC-derived peptides binding to XIAP-BIR3 (Wu et al., (2000) Nature 408, 1008-1012), with only the N-terminal four residues of the peptide in contact with the protein (Figure 5). The N-termini of the peptides are in acidic environments with the amino group of Ala1' hydrogen-bonded to the side-chain carboxylates of Aspartate 138 and Glutamic Acid 143. Peptide residues 2'-4' form a highly twisted extension to the antiparallel β-sheet of ML-IAP-BIR, with hydrogen-bonds between Val/Glu2' amide and Gln132 carbonyl, Val/Glu2' carbonyl and Gln132 amide, and a long (N-O distance ~3.3 Å) hydrogen-bond between Ile/Val4' amide and Gly130 carbonyl. In the phage-derived peptide complexes an additional hydrogen-bond may be present between the side-chain carboxylate of Glu2' and the side-chain hydroxyl of Ser133.

The interactions are further stabilized by hydrophobic contacts between the peptides and ML-IAP-BIR. The methyl group of Ala1' is buried in a hydrophobic pocket formed by the side-chains of Leu131, Trp134, and Glu143. The side-chain of peptide residue 2' (Val/Glu2') makes van der Waals contact with the β-methylene of Ser133 in each of the three complexes. Residue 3' differs in the three complexes and makes different hydrophobic contacts in each case. Pro3' in the SMAC-derived peptide makes van der Waals contact with Trp147 as well as the side-chain of Val2'. The side-chain methyl of Ala3' in the AEAVPWKSE (SEQ ID NO:9) peptide has no significant van der Waals contacts with the protein, while the side-chain of Val3' in AEVVAVKSE (SEQ ID NO:10) is readily accommodated in a hydrophobic groove defined by the side-chains of Trp147 and Phe148. ML-IAP-BIR also tolerates Leu in this position of the peptide, as observed in the ML-IAP intermolecular interactions found in the crystal structures (Figure 2). Finally, the side-chain of peptide residue 4' (Ile/Val4') interacts with a hydrophobic pocket defined by Gly130-Gln132, and the aliphatic portions of Thr116, Lys121, and Arg123. In the complexes with the phage-derived peptides, Lys121 has moved from its position in the SMAC peptide complex to fill the space left vacant by substitution of Ile4' with the smaller Val4', indicating that there is some flexibility in this pocket.

| Table 2: Data collection and refinement statistics[a] | | | | | | |
|---|---|---|---|---|---|---|
| Peptide | AVPIAQKSE * (SEQ ID NO:1) | AEAVPWKSE * (SEQ ID NO:9) MAD (Zn K edge) | | | AEVVAVKSE * (SEQ ID NO: 10) | AVPX24 (SEQ ID NO: 52) |
| Data set | | Peak | inflection | remote | | |
| Wavelength (Å) | 1.2686 | 1.2822 | 1.2834 | 1.1921 | 1.033 | 1.08 |
| Resolution (Å) | 20-2.3 | 20-2.2 | 20-2.25 | 20-2.2 | 30-2.7 | 30-2.3 |
| $R_{sym}$ | 6.5 (37.9) | 4.0 (21.6) | 5.0 (32.4) | 5.0 (28.2) | 13.7 (28.2) | 6.1 (43.1) |
| I/$\sigma$(I) | 21.8 (3.4) | 19.1 (4.4) | 14.2 (2.4) | 14.9 (3.2) | 8.5 (2.6) | 11.8 (1.9) |
| Redundancy | 3.7 (3.9) | 2.0 (2.0) | 2.1 (2.1) | 2.1 (2.1) | 2.1 (2.1) | 1.9 (1.9) |
| Completeness | 97.2(100) | 98.6 (99.6) | 99.4 (99.8) | 99.0 (99.8) | 99.0(99.5) | 98.9 (98.3) |
| MAD phasing | | | 20-3.0 Å | | | |
| Overall FOM | | | 0.75 | | | |
| Refinement | | | | | | |
| Resolution range (Å) | 20-2.3 | 20-2.2 | | | 30-2.7 | 20-2.3 |
| Unique reflections | 59357 | 71265 | | | 32692 | 37505 |
| $R_{cryst}$[b]/$R_{free}$[c] | 17.2/21.4 | 16.7/21.5 | | | 15.0/20.8 | 17.8/22.9 |
| RMSD bonds (Å) | 0.006 | 0.007 | | | 0.012 | 0.008 |
| RMSD angles (°) | 0.87 | 0.91 | | | 1.32 | 1.03 |

(continued)

Table 2: Data collection and refinement statistics[a]

| Peptide | AVPIAQKSE * (SEQ ID NO:1) | AEAVPWKSE * (SEQ ID NO:9) MAD (Zn K edge) | AEVVAVKSE * (SEQ ID NO: 10) | AVPX24 (SEQ ID NO: 52) |
|---|---|---|---|---|
| Ramachandran statistics | | | | |
| most favored (%) | 86.6 | 86.9 | 85.6 | 87.8 |
| additional allowed (%) | 12.4 | 12.1 | 13.4 | 11.2 |

* these peptides are listed for illustrative purposes only.

[a] In each case the space group and unit cell are P3$_2$, $a$ = 83.8, $b$ = 83.8, $c$ = 94.3. Numbers in parentheses are statistics for the highest resolution shell. All data were scaled keeping Bijvoet mates separate. The AVPIAQKSE (SEQ ID NO:1) structure has five molecules in the asymmetric unit, with a total of 3997 protein atoms, 368 waters, 5 zinc atoms, and one heptaethylene glycol molecule. The other structures also have five molecules in the asymmetric unit and almost identical numbers of atoms.

$$^{b} \quad R_{cryst} = \Sigma|(F\mathrm{obs}) - (F\mathrm{calc})|/\Sigma(F\mathrm{obs}).$$

[c] R$_{free}$ is defined similarly to R$_{cryst}$ but comprises a test set of 5% of the total reflections that were not used in model refinement.

## EXAMPLE 11 Positional Scanning

The results of the phage sorting and subsequent structural analysis of the ML-IAP-BIR/peptide complexes suggest that peptide modifications could be made that would increase binding affinity and selectivity for ML-IAP relative to XIAP-BIR3. In order to examine the effects of substitutions at positions 2', 3', and 4' in a more systematic manner, a series of single point mutants were synthesized in the background of the SMAC-based peptide, AVPIAQKSE (SEQ ID NO:1), and measured for binding to both ML-IAP-BIR and XIAP-BIR3. The $K_i$ values for the mutant peptides relative to the $K_i$ of AVPIAQKSE (SEQ ID NO:1) binding to ML-IAP-BIR are plotted in Figure 6, with Ki values for peptides binding to ML-IAP-BIR, XIAP-BIR2 and XIAP-BIR3 in Table 3. The importance of these residues for binding to both ML-IAP-BIR and XIAP-BIR3 is emphasized by the greater than 10-fold losses in affinity observed upon substitution with Alanine. Of the other natural amino acid substitutions, only Phe and Trp at position 4' result in increased affinity relative to the SMAC-based peptide.

Several mutations result in improved specificity for ML-IAP-BIR relative to XIAP-BIR3. In particular, mutant peptides with Glu or Asp at position 2' are 7-8-fold selective for ML-IAP-BIR. Substitution of Pro3' with either Val, Ile, or Leu results in > 10-fold specificity for ML-IAP-BIR. Structure-based modeling suggested that substitution of Pro3' with (2S, 3S)-3-methylpyrrolidine-2-carboxylic acid [(3S)-methyl-proline] would result in a peptide with improved affinity for ML-IAP-BIR relative to the Val, Ile, or Leu substituted peptides, while maintaining the specificity advantage imparted by these amino acids. The resulting peptide has 7-fold greater affinity for ML-IAP-BIR than the starting SMAC-based peptide ($K_i$ = 70 nM compared to 0.5 µM), and is ~100-fold specific for ML-IAP-BIR relative to XIAP-BIR3.

A number of non-natural amino acid substitutions at position 4' also result in improved specificity for ML-IAP-BIR. The greatest specificity enhancement is observed with homophenylalanine (X12) (-7-fold), followed by 2-naphthyl-lalanine (X1), 4-amino-phenylalanine (X4), and 4-phenyl-phenylalanine (X14). Of these substitutions, only 2-naphthylalanine has a significantly reduced affinity for ML-IAP-BIR, relative to the SMAC-based peptide (-3-fold reduced), while the binding affinity for XIAP-BIR3 is reduced in all four cases.

Table 3: $K_i$ values for positional scanning peptide homologues

| Peptide Sequence | $K_i$ (µM) | | |
|---|---|---|---|
| | ML-IAP-BIR | XIAP-BIR3 | XIAP-BIR2 |
| AVPIAQKSE | 0.50 | 0.67 | 13.8 |

(continued)

Table 3: $K_i$ values for positional scanning peptide homologues

| Peptide Sequence | $K_i$ (µM) | | |
|---|---|---|---|
| | ML-IAP-BIR | XIAP-BIR3 | XIAP-BIR2 |
| (SEQ ID NO:1)<br>A**G**PIAQKSE | 23.9 | 31.2 | >300 |
| (SEQ ID NO:12)<br>A**A**PIAQKSE | 8.0 | 10.1 | 122 |
| (SEQ ID NO:13)<br>A**I**PIAQKSE | 0.66 | 0.73 | 30.5 |
| (SEQ ID NO:14)<br>A**L**PIAQKSE | 1.7 | 2.9 | >300 |
| (SEQ ID NO:15)<br>A**F**PIAQKSE | 0.99 | 2.1 | 38.1 |
| (SEQ ID NO:16)<br>A**Y**PIAQKSE | 1.6 | 1.5 | 26.7 |
| (SEQ ID NO:17)<br>A**W**PIAQKSE | 1.9 | 1.4 | 45.8 |
| (SEQ ID NO:18)<br>A**P**PIAQKSE | >230 | >415 | >300 |
| (SEQ ID NO:19)<br>A**S**PIAQKSE | 2.8 | 3.0 | 38.1 |
| (SEQ ID NO:20)<br>A**T**PIAQKSE | 2.8 | 4.2 | 45.8 |
| (SEQ ID NO:21)<br>A**M**PIAQKSE | 1.1 | 1.8 | 14.5 |
| (SEQ ID NO:22)<br>A**N**PIAQKSE | 9.0 | 2.0 | 68.7 |
| (SEQ ID NO:23)<br>A**Q**PIAQKSE | 1.3 | 3.2 | 30.5 |
| (SEQ ID NO:24)<br>A**D**PIAQKSE | 7.0 | 56.9 | 53.4 |
| (SEQ ID NO:25)<br>A**E**PIAQKSE | 1.8 | 13.1 | 30.5 |
| (SEQ ID NO:26)<br>A**H**PIAQKSE | 2.4 | 0.83 | 38.1 |
| (SEQ ID NO:27)<br>A**K**PIAQKSE | 1.9 | 1.2 | 83.9 |
| (SEQ ID NO:28)<br>A**R**PIAQKSE | 0.73 | 0.77 | 68.7 |
| (SEQ ID NO:29)<br>AV**A**IAQKSE | 14.6 | 25.5 | 20.1 |
| (SEQ ID NO:30)<br>AV**V**IAQKSE | 2.1 | 35.3 | 38.6 |
| (SEQ ID NO:31)<br>AV**I**IAQKSE | 3.5 | 36.1 | 44.2 |
| (SEQ ID NO:32)<br>AV**L**IAQKSE | 3.5 | 114.0 | 83.9 |
| (SEQ ID NO:33)<br>AV**X**IAQKSE[a]<br>(SEQ ID NO:34) | 0.07 | 7.2 | 22.1 |

(continued)

Table 3: $K_i$ values for positional scanning peptide homologues

| Peptide Sequence | $K_i$ (μM) | | |
|---|---|---|---|
| | ML-IAP-BIR | XIAP-BIR3 | XIAP-BIR2 |
| AVP**A**AQKSE (SEQ ID NO:35) | 8.2 | 25.5 | 71.2 |
| AVP**F**AVKSE (SEQ ID NO:36) | 0.16 | 0.39 | 14.7 |
| AVP**Y**AQKSE (SEQ ID NO:37) | 0.79 | 1.0 | 16.9 |
| AVP**X1**AQKSE[a] (SEQ ID NO:38) | 1.3 | 5,3 | 55.2 |
| AVP**X2**AQKSE[a] (SEQ ID NO:39) | 2.8 | 8.5 | 30.5 |
| AVP**X3**AQKSE[a] (SEQ ID NO:40) | 1.0 | 1.3 | 51.6 |
| AVP**X4**AQKSE[a] (SEQ ID NO:41) | 0.55 | 2.0 | 71.7 |
| AVP**X5**AQKSE[a] (SEQ ID NO:42) | 0.52 | 0.70 | 13.3 |
| AVP**X6**AQKSE[a] (SEQ ID NO:43) | 2.7 | | 122 |
| AVP**X7**AQKSE[a] (SEQ ID NO:44) | 7.9 | 14.5 | 229 |
| AVP**X9**AQKSE[a] (SEQ ID NO:45) | 0.87 | 2.0 | 35.1 |
| AVP**X10**AQKSE[a] (SEQ ID NO:46) | 0.45 | 1.0 | 27.7 |
| AVP**X12**AQKSE[a] (SEQ ID NO:47) | 0.63 | 4.6 | 6.7 |
| AVP**X13**AQKSE[a] (SEQ ID NO:48) | 1.3 | 2.1 | 122 |
| AVP**X14**AQKSE[a] (SEQ ID NO:49) | 0.55 | 1.77 | 16.9 |

all peptides are listed for illustrative purposes only.

[a] Nonnatural amino acids are indicated as follows: X, (3S)-methyl-proline; X1, 2-naphthylalanine; X2, phenylalanine-4-sulfonic acid; X3,4-nitro-phenylalanine; X4, 4-amino-phenylalanine; X5, 3-methoxy-phenylalanine; X6, cyclohexylalanine; X7, cyclopentylalanine; X9, 3,5-dibromo-tyrosine; X10, 4-iodo-phenylalanine; X12, homophenylalanine; X13, 4-ketophenyl-phenylalanine; X14, 4-phenyl-phenylalanine.

EXAMPLE 12: Phenylethylamine scan.

A series of phenethylamine derivatives (Table 4) were synthesized by either manual or automated (Quest™) synthesis. The phenethylamine was reductively aminated to ArgoGel-MB-CHO™ resin (0.45 mmol/g) using sodium cyanoborohydride (3 equiv.) in 2% acetic acid in N-methylpyrrolidinone for 12 hours. The amino acids alanine, valine, and proline were added using standard Fmoc chemistry. The phenethylamine peptide was cleaved from the resin using trifluoroacetic acid and purified using HPLC with an $H_2O$-acetonitrile gradient with 0.1 % trifluoroacetic acid. Masses of each compound were verified by electrospray mass spectrometry.

The phage-display data, peptide alanine-scan data (data not shown), and X-ray crystal structures of ML-IAP-BIR/peptide complexes indicate that the four N-terminal peptide residues are sufficient for high-affinity binding to ML-IAP-BIR, consistent with previous results for XIAP-BIR3 [Wu et al., (2000) Nature 408, 1008-1012, Liu et al., (2000) Nature 408, 1004-1008, Kipp et al.,. (2002) Biochemistry 41, 7344-7349.]

Inspection of the crystal structures also suggests that the C-terminal carboxylate moieties of such four-residue peptides do not contribute significantly to peptide binding. In order to further explore the effects of substitution at position 4' a series of compounds in which this amino acid is substituted with a selection of phenylethylamine derivatives were synthesized and compared to the peptides AVPI and AVPF for binding to ML-IAP-BIR and XIAP-BIR3. The $K_i$ values for these compounds relative to the $K_i$ of AVPI binding to ML-IAP-BIR are plotted in Figure 7 ($K_i$ values for these compounds binding to ML-IAP-BIR, XIAP-BIR2 and XIAP-BIR3 are also reported in Table 4).

As noted previously for the P4 amino acid scan in the context of the nine-residue SMAC-based peptide, substitution of Ile4' with Phe in the context of the four-residue peptide results in a significant improvement in binding affinity for both ML-IAP-BIR and XIAP-BIR3. Substitution with (S)-2-amino-3-phenyl-1-propanol (X32) or (S)-α-(methoxymethyl)-phenylethylamine (X38), which are structurally very similar to Phe, results in binding affinities that are similar to those found for the four-residue peptide AVPF. In addition, substitution with several of the phenylethylamine derivatives result in improved specificity for ML-IAP-BIR. The greatest specificity enhancement is observed with 2,2-diphenylethylamine (X24) (-9-fold), followed by trans-(1R,2S)-2-phenylcyclopropyl-1-amine (X28a), and (1R,2S)-norephedrine (X29). Of these substitutions, 2,2-diphenylethylamine results in significantly higher affinity for ML-IAP-BIR than the peptides AVPI (~10-fold improvement) or AVPF (~3-fold improvement).

In order to understand more fully the specificity and affinity improvements afforded by substitution of peptide residue 4' with 2,2-diphenylethylamine, ML-IAP-BIR was crystallized in complex with AVPX24 using protocols similar to those used for the SMAC-based and phage-derived peptide complexes. Data for the ML-IAP-BIR/AVPX24 complex were collected at beamline 9-1 of the Stanford Synchrotron Radiation Laboratory. The previously determined structure of ML-IAP-BIR from the AEAVPWKSE (SEQ ID NO:9) complex (but without the peptide) was used to generate Fo - Fc difference electron density maps, into which the AVPX24 molecule could be placed easily. The position of the two phenyl rings of the 2,2-diphenylethylamine moiety was unambiguous. The complex was then subjected to one round of positional and individual atomic B-factor refinement using Refmac.

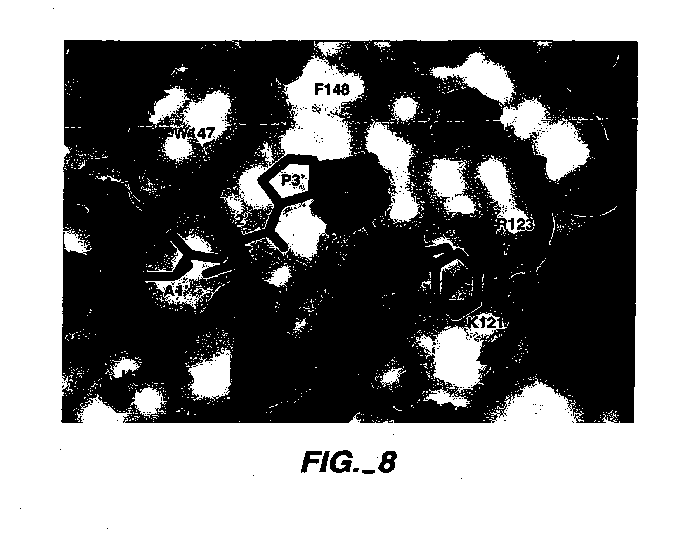

The structure was determined to a resolution of 2.3 Å (Table 2). The bound conformations of Ala1', Val2', and Pro3' in AVPX24 are almost identical to those seen in the ML-IAP-BIR/AVPIAQKSE (SEQ ID NO:1) complex (Figure 8). One of the phenyl rings of the 2,2-diphenylethylamine moiety packs into the hydrophobic P4 pocket where it makes extensive contacts with protein residues Thr116, Lys121-Arg123, and Gly130-Gln132. In contrast, the side-chain of Ile4' in the ML-IAP-BIR/AVPIAQKSE (SEQ ID NO:1) complex contacts protein residues Lys121, and Gly130-Gln132, only. The second phenyl ring of the 2,2-diphenylethylamine moiety packs more on the surface of the protein with one edge contacting the hydrophobic portion of the side-chain of Lys121. The 10-fold increase in affinity for ML-IAP-BIR observed upon substitution of Ile4' in AVPI with 2,2-diphenylethylamine in AVPX24 can thus be explained by the additional hydrophobic contacts observed for the two phenyl rings in the latter complex. Similarly, the 3-fold increase in affinity relative to AVPF appears to result from the additional hydrophobic contacts between the second phenyl ring and the protein.

| Table 4: $K_i$ values for P4 amino acid/phenylethylamine scan | | | |
|---|---|---|---|
| Peptide * Sequence[a] | $K_i$ (μM) | | |
| | ML-IAP-BIR | XIAP-BIR3 | XIAP-BIR2 |
| AVPI (SEQ ID NO:50) | 0.33 | 1.7 | 9.6 |
| AVPF (SEQ ID NO:51) | 0.09 | 0.22 | 6.5 |
| AVP**X24** (SEQ ID NO:52) | 0.03 | 0.28 | 6.4 |
| AVP**X25** (SEQ ID NO:52) | 1.5 | 7.2 | 13.0 |
| AVP**X26** (SEQ ID NO:52) | 1.9 | 1.2 | 6.4 |
| AVP**X27** (SEQ ID NO:52) | 0.41 | 1.6 | 5.5 |
| AVP**X28a** (SEQ ID NO:52) | 0.85 | 7.6 | N.D. |
| AVP**X28b** (SEQ ID NO:52) | 1.0 | 1.4 | N.D. |

(continued)

| Peptide * Sequence[a] | $K_i$ (μM) | | |
|---|---|---|---|
| | ML-IAP-BIR | XIAP-BIR3 | XIAP-BIR2 |
| AVP**X29** (SEQ ID NO:52) | 0.99 | 7.6 | 11.0 |
| AVP**X31** (SEQ ID NO:52) | 0.27 | 0.69 | 4.7 |
| AVP**X32** (SEQ ID NO:52) | 0.09 | 0.39 | 441 |
| AVP**X33** (SEQ ID NO:52) | 0.31 | 0.85 | 9.4 |
| AVP**X34** (SEQ ID NO:52) | 0.78 | 1.1 | 5.3 |
| AVP**X36** (SEQ ID NO:52) | 1.3 | 3.3 | 11.9 |
| AVP**X37** (SEQ ID NO:52) | 0.87 | 0.84 | 7.3 |
| AVP**X38** (SEQ ID NO:52) | 0.15 | 0.46 | 6.8 |
| AVP**X39** (SEQ ID NO:52) | 0.68 | 0.68 | 3.8 |
| AVP**X40** (SEQ ID NO:52) | 0.30 | 0.32 | 6.3 |

Table 4: $K_i$ values for P4 amino acid/phenylethylamine scan

[a] Phenylethylamine derivatives are indicated as follows: X24, 2,2-diphenylethylamine; X25, (1S,2S)-(+)-2-amino-1-phenyl-1,3-propandiol; X26, 3-trifluoromethylphenylethylamine; X27, (1R,2R)-(-)-2-amino-1-phenyl-1,3-propandiol; X28a, trans-(1R,2S)-phenylcyclopropyl-1-amine; X28b, trans-(1S,2R)-2-phenylcyclopropyl-1-amine; X29, (1R, 1S)-(+)-norephedrine; X31, β-methylphenylethylamine; X32, (S)-(-)-2-amino-3-phenyl-1-propanol; X33, (R)-(-)-2-amino-1-phenylethanol; X34, 3-ethoxyphenylethylamine; X36, 5-bromo-2-methoxyphenylethylamine; X37, 3-fluorophenylethylamine; X38, (S)-(+)-α-(methoxymethyl)-phenylethylamine; X39, 3-chlorophenylethylamine; X40, 2-ethoxyphenylethylamine. All data (except for AVPX24) were scaled keeping Bijvoet mates separate.

* these peptides are listed for illustrative purposes only.

EXAMPLE: 13: Dipeptide Isostere scan:

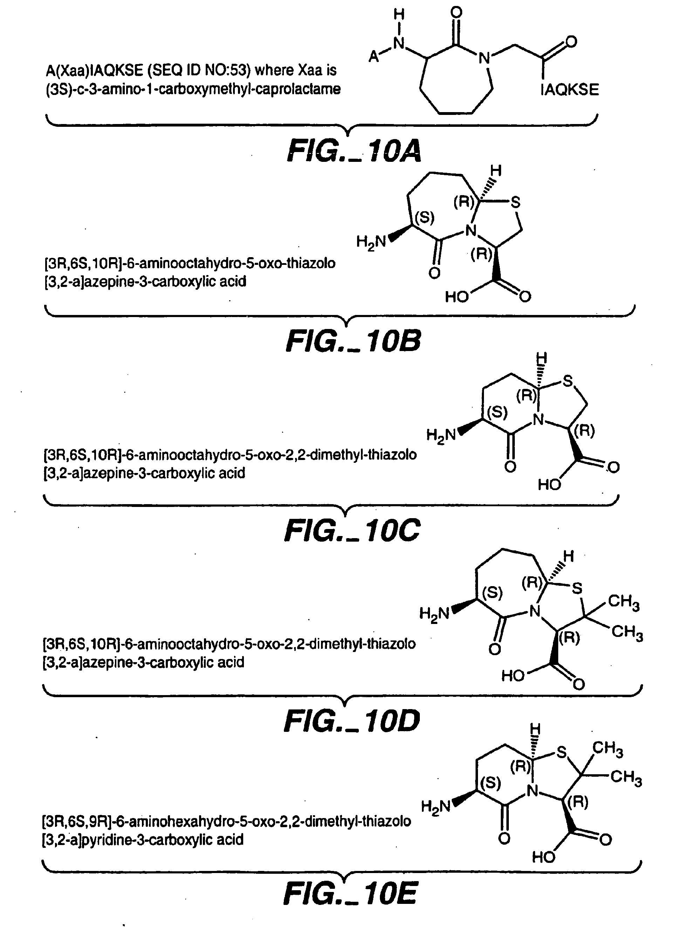

Molecular modeling studies based on the crystal structures of the ML-IAP-BIR/peptide complexes suggested that Va12' and Pro3' of the Smac-based peptide, AVPIAQKSE (SEQ ID NO:1), could be replaced with dipeptide isosteres that would maintain the main-chain to main-chain hydrogen-bonds observed between Val2' and Gln132 of the protein. To test this hypothesis the compound shown in Figure 10A was synthesized.

The IAQKSE amino acid sequence was synthesized on resin using standard Fmoc chemistry. (3S)-Fmoc-3-amino-1-carboxymethyl-caprolactame (Neosystem™) was added to this sequence using PyBop coupling (12 hours). Alanine was added using standard peptide chemistry and the peptide was cleaved from the resin using trifluoroacetic acid and purified using HPLC with an $H_2O$-acetonitrile gradient with 0.1% trifluoroacetic acid. This resulted in the sequence A(Xaa)IAQKSE (SEQ ID NO:53), where Xaa is one of the dipeptide isosteres described in this Example. Masses of each compound were verified by electrospray mass spectrometry. The resulting compound was tested for binding to ML-IAP-BIR and XIAP-BIR3 using a fluorescence polarization-based competition assay with 5-carboxyfluorescein-conjugated Hid peptide [AVPFAK(5-FAM)K, Hid-FAM] as the probe. The $K_i$ values were 15.3 and 39.8 μM for binding to ML-IAP-BIR and XIAP-BIR3, respectively. Peptides containing such dipeptide isosteres are thus capable of binding to and antagonizing the IAP proteins. The -30-fold loss in affinity observed for this compound binding to ML-IAP-BIR relative to the Smac-based peptide, AVPIAQKSE (SEQ ID NO:1), likely results in part from loss of contacts between the side-chain of Pro3' and the protein. Related dipeptide isosteres, such as [3R,6S,10R]-6-aminooctahydro-5-oxo-thiazolo[3,2-a]azepine-3-carboxylic acid or [3R,6S,9R]-6-aminohexahydro-5-oxo-thiazolo[3,2-a]pyridine-3-carboxylic acid, are thus expected to have improved affinity for binding to ML-IAP-BIR by reintroducing contacts similar to those observed between Pro3" of the Smac-based peptide, AVPIAQKSE (SEQ ID NO:1), and the protein. Structure-based modeling further suggests that introduction of methyl groups in these dipeptide isosteres, as in [3R,6S,10R]-6-aminooctahydro-5-oxo-2,2-dimethyl-thiazolo[3,2-a]azepine-3-carboxylic acid, or [3R,6S,9R]-6-aminohexahydro-5-oxo-2,2-dimethyl-thiazolo [3,2-a]pyridine-3-carboxylic acid, as shown in Figure 10B- 10E, would result in further improved affinity for ML-IAP-BIR and increased specificity for ML-IAP-BIR relative to XIAP-BIR3 (as did substitution of Pro3' with (3S)-methyl-proline in the Smac-based peptide, AVPIAQKSE (SEQ ID NO:1) as described in EXAMPLE 11.

EXAMPLE 14: ML-IAP Mutations for screening.

Nuclear magnetic resonance (NMR)-based methods might be used to identify compounds that bind weakly to ML-IAP-BIR, and to aid their development into more potent antagonists that could be used as lead compounds in the drug discovery process. In particular, the SAR-by-NMR (structure-activity relationship by NMR) method, and variations thereof, have been widely applied to drug discovery NMR [Shuker et al., (1996) Science 274, 1531-1534.]

Such methods are based on the use of protein chemical-shift changes to identify low-affinity ligands that target relevant binding sites on the protein. A prerequisite of such chemical-shift mapping methods is reasonable resolution, and preferably sequence-specific assignments, of protein resonances in two-dimensional heteronuclear-correlation spectra (either $^{15}N,^{1}H$- or $^{13}C,^{1}H$-correlation spectra). Unfortunately, the ML-IAP-BIR domain aggregates significantly in the concentration range required for NMR spectroscopy, resulting in poor quality NMR spectra that preclude the use of protein chemical-shift mapping methods to identify low-affinity ligands.

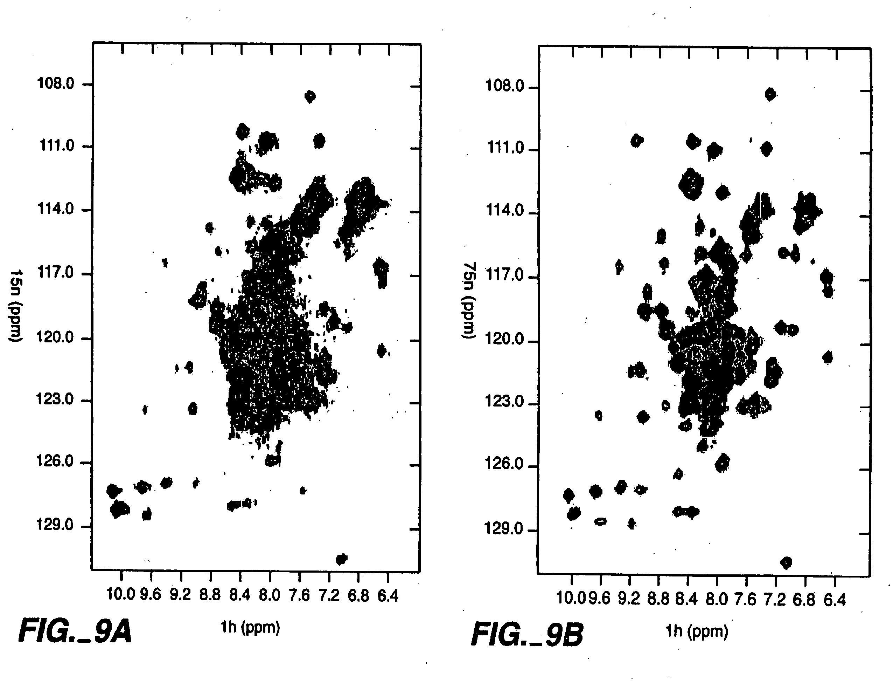

However, inspection of the asymmetric unit of the crystals of ML-IAP-BIR reveals a hydrophobic interface within the tetramer formed by protomers A-D, that might be responsible for the aggregation observed in the solution phase. Substitution of some of the hydrophobic amino acids within the interface identified by the X-ray crystal structure with hydrophilic amino acids would, thus, be expected to reduce the solution aggregation and consequently improve the quality of the NMR spectra of ML-IAP-BIR. To test this hypothesis, ML-IAP-BIR residues Phe81 and Leu89 (Figure 2) were mutated to Glu and Asp, respectively. Comparison of the $^{15}N,^{1}H$-heteronuclear single-quantum coherence (HSQC) spectra of wild-type and Phe81Glu/Leu89Asp mutant ML-IAP-BIR proteins shows that the spectral quality can be improved dramatically (Figure 9) by mutating residues found in the tetramer-interface. Such mutations result in ML-IAP-BIR variants that are amenable to NMR-based screening methods.

EXAMPLE 15: Apoptosis Assay.

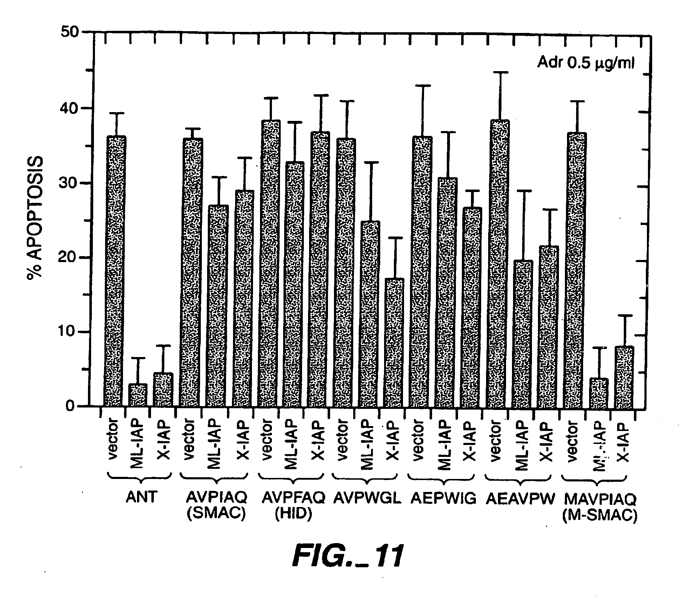

MCF7 cells were transiently transfected with 0.2 μg of the reporter plasmid pCMV-pgal plus 0.2 μg of plasmid encoding Fas, TNFR1, DR4 or DR5 and 1.6 μg of plasmid encoding vector, baculovirus P35 or human IAPs. For adriamycin or 4-TBP treatment, MCF7 cells were transiently transfected with the reporter plasmid pCMV-βgal and control vector or IAPs. Four hours following transfection, adriamycin (doxorubicin, Sigma) or 4-TBP (Aldrich) was added to the media at the indicated concentrations. Apoptosis was assayed 16 hours later as described in Pan et al., (1997) Science 277:815-818. Melanoma cell lines (888, 624) or melanocytes (NHEM cells) were treated for 5 hours with the indicated amounts of 4-TBP and viability assessed by FACS analysis using staining with propidium iodide and annexin V (Clontech). Using this assay, it was found that a SMAC peptide could block the anti-apoptotic activity of ML-IAP (Vucic et al., (2002) J.Biol. Chem. 277; 12275-12279). SMAC-like peptides block the anti-apoptotic activity of ML-IAP. MCF7 cells were transiently transfected with 0.15 mg of the reporter plasmid pCMV-βgal β-galactosidase) and 0.85 mg of either plasmid encoding vector alone, ML-IAP or X-IAP. Following transfection, SMAC-antennapedia (penetratin; RQIKIWFQNRRMKWKK-NH2 (SEQ ID NO:54 )) fusion peptide or other indicated peptide-antennapedia fusions (50 mM) were added where shown and three hours later cells were exposed to adriamycin (0.5 mg/ml). Twenty-four hours after transfection, cells were stained with 5-bromo-4-chloro-3-indoxyl-b-D-galactopyranoside (X-Gal) and examined for their morphology. The data (average ± standard deviation) represent the percentage of round, apoptotic cells as a function of total β-galactosidase-positive cells (n=3). This data is shown in Figure 11.

**Claims**

1. An isolated BIR Binding Domain (BDB) oligopeptide, of sequence $AN_2N_3N_4$, wherein;
   $N_2$ is Glu or Asp
   $N_3$ is Val, Ile, Leu or (2S, 3S)-3-methylpyrrolidine-2-carboxylic acid [(3S)-methyl -proline]
   $N_4$ is homophenylalanine, 4-amino-phenylalanine, 4-phenyl-phenylalanine, 2,2-diphenylethylamine, (1S,2s)-(+)-2-amino-1-phenyl-1,3-propandiol, 3-trifluoromethylphenylethylamine, (1R,2R)-(-)-2-amino-1-phenyl-1,3-propandiol, trans-2-phenylcyclopropylamine, (1R,1S)-(+)-norephedrine, β-methylphenylethylamine, (S)-(-)-2-amino-3-phenyl-1-propanol, (R)-(-)-2-amino-1-phenylethanol, 3-ethoxyphenylethylamine, 5-bromo-2-methoxyphenylethylamine, 3-fluorophenylethylamine, (S)-(+)-α-(methoxymethyl)-phenylethylamine, 3-chlorophenylethylamine, or 2-ethoxyphenylethylamine.

**2.** The BDB oligopeptide of Claim 1 further comprising a dipeptide isostere.

**3.** The BDB oligopeptide of Claims 1 or Claim 2 fused to a heterologous sequence that transports it across a cell membrane.

**4.** The BDB oligopeptide of any one of Claims 1-3 which is conjugated to a cytotoxic agent.

**5.** The BDB oligopeptide of Claim 4, wherein the cytotoxic agent is selected from the group consisting of toxins, antibiotics, radioactive isotopes and nucleolytic enzymes.

**6.** The BDB oligopeptide of Claim 5 which induces apoptosis when administered to a cell.

**7.** A method of increasing apoptosis in a cell comprising; contacting said cell *in vitro* with an effective amount of the oligopeptide of any one of Claims 1-4, wherein said apoptosis is increased.

**8.** The BDB oligopeptide of any one of Claims 1-6 for use in a method of treatment to increase apoptosis in a cell of a mammal.

**9.** Use of a BDP oligopeptide according to any one of Claim 1-6 in the manufacture of a medicament for increasing apoptosis in a cell of a mammal.

**10.** The method of Claim 7. oligopeptide of Claim 8 or use according to Claim 9 wherein said cell is a cancer cell.

**11.** The method, oligopeptide or use according to Claim 10, wherein said cancer cell is selected from the group consisting of a melanoma cell, a breast cancer cell, a colorectal cancer cell, a lung cancer cell, an ovarian cancer cell, a central nervous system cancer cell, a liver cancer cell, a bladder cancer cell, a pancreatic cancer cell, a cervical cancer cell, and a leukemia cell.

**12.** The oligopeptide of Claim 8 or use according to Claim 9, wherein the oligopeptide or medicament is for administration with a second cytotoxic agent.

**13.** The oligopeptide of Claim 8 or use according to Claim 9, wherein the oligopeptide or medicament is for administration with APO2/TRAIL polypeptide.

**14.** The oligopeptide or use according to Claim 12, wherein said second cytotoxic agent is adriamycin (doxorubicin), 4-tertiary butylphenol, etoposide, taxol, camptothecin, methotrexate, vincristine, tamoxifen, BCNU, streptozoicin, vincristine, 5-fluorouracil or esperamicins.

**15.** The BDB oligopeptide of any one of Claims 1-4 in admixture with a carrier.

**16.** The oligopeptide of Claim 15, wherein said carrier is a pharmaceutically acceptable carrier.

**17.** An article of manufacture comprising:

(a) a container; and
(b) the BDB oligopeptide of any one of Claims 1-5 contained within said container
(c) a label affixed to said container, or a package insert included with said container, referring to the use of said oligopeptide for the therapeutic treatment of or the diagnostic detection of a cancer.

**Patentansprüche**

**1.** Isoliertes BIR-Bindungsdomänen- (BDB-) Oligopeptid der Sequenz $AN_2N_3N_4$, worin:

$N_2$ Glu oder Asp ist,

N$_3$ Val, Ile, Leu oder (2S,3S)-3-Methylpyrrolidin-2-carbonsäure [(3S)-Methylprolin] ist,

N$_4$ Homophenylalanin, 4-Aminophenylalanin, 4-Phenylphenylalanin, 2,2-Diphenylethylamin, (1S,2S)-(+)-2-Amino-1-phenyl-1,3-propandiol, 3-Trifluormethylphenylethylamin, (1R,2R)-(-)-2-Amino-1-phenyl-1,3-propandiol, trans-2-Phenylcyclopropylamin, (1R,1S)-(+)-Norephedrin, β-Methylphenylethylamin, (S)-(-)-2-Amino-3-phenyl-1-propanol, (R)-(-)-2-Amino-1-phenylethanol, 3-Ethoxyphenylethylamin, 5-Brom-2-methoxyphenylethylamin, 3-Fluorphenylethylamin, (S)-(+)-α-(Methoxymethyl)phenylethylamin, 3-Chlorphenylethylamin oder 2-Ethoxyphenylethylamin ist.

2. BDB-Oligopeptid nach Anspruch 1, weiters umfassend ein Dipeptid-Isoster.

3. BDB-Oligopeptid nach Anspruch 1 oder Anspruch 2, fusioniert an eine heterologe Sequenz, die es durch eine Zellmembran transportiert.

4. BDB-Oligopeptid nach einem der Ansprüche 1 bis 3, das an ein zytotoxisches Mittel konjugiert ist.

5. BDB-Oligopeptid nach Anspruch 4, worin das zytotoxische Mittel aus der aus Toxinen, Antibiotika, radioaktiven Isotopen und nucleolytischen Enzymen bestehenden Gruppe ausgewählt ist.

6. BDB-Oligopeptid nach Anspruch 5, das bei Verabreichung an eine Zelle Apoptose induziert.

7. Verfahren zur Verstärkung von Apoptose in einer Zelle, umfassend das Kontaktieren der Zelle in vitro mit einer wirksamen Menge des Oligopeptids nach einem der Ansprüche 1 - 4, worin die Apoptose verstärkt wird.

8. BDB-Oligopeptid nach einem der Ansprüche 1 - 6 zur Verwendung in einem Behandlungsverfahren, um Apoptose in einer Zelle eines Säugetiers zu verstärken.

9. Verwendung eines BDB-Oligopeptids nach einem der Ansprüche 1 - 6 bei der Herstellung eines Medikaments zur Verstärkung von Apoptose in einer Zelle eines Säugetiers.

10. Verfahren nach Anspruch 7, Oligopeptid nach Anspruch 8 oder Verwendung nach Anspruch 9, worin es sich bei der Zelle um eine Krebszelle handelt.

11. Verfahren, Oligopeptid oder Verwendung nach Anspruch 10, worin die Krebszelle aus der aus einer Melanom-Zelle, einer Brustkrebs-Zelle, einer kolorektalen Krebszelle, einer Lungenkrebs-Zelle, einer Eierstockkrebs-Zelle, einer Krebszelle des Zentralnervensystems, einer Leberkrebs-Zelle, einer Blasenkrebs-Zelle, einer Pankreas-Krebszelle, einer Zervix-Krebszelle und einer Leukämiekrebs-Zelle bestehenden Gruppe ausgewählt ist.

12. Oligopeptid nach Anspruch 8 oder Verwendung nach Anspruch 9, worin das Oligopeptid oder Medikament zur Verabreichung mit einem zweiten zytotoxischen Mittel gedacht ist.

13. Oligopeptid nach Anspruch 8 oder Verwendung nach Anspruch 9, worin das Oligopeptid oder Medikament zur Verabreichung mit APO2/TRAIL-Polypeptid dient.

14. Oligopeptid oder Verwendung nach Anspruch 12, worin es sich bei dem zweiten zytotoxischen Mittel um Adriamycin (Doxorubicin), 4-tert-Butylphenol, Etoposid, Taxol, Camptothecin, Methotrexat, Vincristin, Tamoxifen, BCNU, Streptozoicin, Vincristin, 5-Fluoruracil oder Esperamicine handelt.

15. BDB-Oligopeptid nach einem der Ansprüche 1 - 4 in Beimischung mit einem Träger.

16. Oligopeptid nach Anspruch 15, worin es sich bei dem Träger um einen pharmazeutisch annehmbaren Träger handelt.

17. Fertigartikel, umfassend:

(a) einen Behälter; sowie
(b) das BDB-Oligopeptid nach einem der Ansprüche 1 - 5, enthalten im Behälter,
(c) eine Markierung, die an dem Behälter fixiert ist, oder ein Beipackzettel, der dem Behälter beigelegt ist, die/der sich auf die Verwendung des Oligopeptids zur therapeutischen Behandlung oder zum diagnostischen Nachweis von Krebs bezieht.

**Revendications**

1. Oligopeptide de domaine de liaison de BIR (BDB) isolé, ayant la séquence $AN_2N_3N_4$, dans laquelle :

   $N_2$ représente Glu ou Asp ;
   $N_3$ représente Val, Ile, Leu ou l'acide (2S,3S)-3-méthylpyrrolidine-2-carboxylique [(3S)-méthyl-proline] ;
   $N_4$ représente l'homophénylalanine, la 4-amino-phénylalanine, la 4-phényl-phénylalanine, la 2,2-diphényléthylamine, le (1S,2S)-(+)-2-amino-1-phényl-1,3-propanediol, la 3-trifluorométhylphényléthylamine, le (1R,2R)-(-)-2-amino-1-phényl-1,3-propanediol, la trans-2-phénylcyclopropylamine, la (1R,1S)-(+)-noréphédrine, la β-méthylphényléthylamine, le (S)-(-)-2-amino-3-phényl-1-propanol, le (R)-(-)-2-amino-1-phényléthanol, la 3-éthoxyphényléthylamine, la 5-bromo-2-méthoxyphényléthylamine, la 3-fluorophényléthylamine, la (S)-(+)-α-(méthoxyméthyl)-phényléthylamine, la 3-chlorophényléthylamine ou la 2-éthoxyphényléthylamine.

2. Oligopeptide de BDB suivant la revendication 1, comprenant en outre un isostère dipeptidique.

3. Oligopeptide de BDB suivant la revendication 1 ou la revendication 2, fusionné à une séquence hétérologue qui le transporte à travers une membrane cellulaire.

4. Oligopeptide de BDB suivant l'une quelconque des revendications 1 à 3, qui est conjugué avec un agent cytotoxique.

5. Oligopeptide de BDB suivant la revendication 4, dans lequel l'agent cytotoxique est choisi dans le groupe consistant en des toxines, des antibiotiques, des isotopes radioactifs et des enzymes nucléolytiques.

6. Oligopeptide de BDB suivant la revendication 5, qui induit une apoptose lors de l'administration à une cellule.

7. Méthode pour augmenter l'apoptose dans une cellule, comprenant : la mise en contact de ladite cellule *in vitro* avec une quantité efficace de l'oligopeptide de l'une quelconque des revendications 1 à 4, dans laquelle ladite apoptose est accrue.

8. Oligopeptide de BDB suivant l'une quelconque des revendications 1 à 6, destiné à être utilisé dans une méthode de traitement pour augmenter l'apoptose dans une cellule de mammifère.

9. Utilisation d'un oligopeptide de BDB suivant l'une quelconque des revendications 1 à 6, dans la production d'un médicament destiné à augmenter l'apoptose dans une cellule d'un mammifère.

10. Méthode suivant la revendication 7, oligopeptide suivant la revendication 8 ou utilisation suivant la revendication 9, où ladite cellule est une cellule cancéreuse.

11. Méthode, oligopeptide ou utilisation suivant la revendication 10, où ladite cellule cancéreuse est choisie dans le groupe consistant en une cellule de mélanome, une cellule de cancer du sein, une cellule de cancer colorectal, une cellule de cancer du poumon, une cellule de cancer des ovaires, une cellule de cancer du système nerveux central, une cellule de cancer du foie, une cellule de cancer de la vessie, une cellule de cancer du pancréas, une cellule de cancer du col de l'utérus et une cellule leucémique.

12. Oligopeptide suivant la revendication 8 ou utilisation suivant la revendication 9, dans lequel l'oligopeptide ou le médicament est destiné à l'administration avec un second agent cytotoxique.

13. Oligopeptide suivant la revendication 8 ou utilisation suivant la revendication 9, dans lequel l'oligopeptide ou le médicament est destiné à l'administration avec un polypeptide AP02/TRAIL.

14. Oligopeptide ou utilisation suivant la revendication 12, dans lequel ledit second agent cytotoxique est l'adriamycine (doxorubicine), le 4-tertiobutylphénol, l'étoposide, le taxol, la camptothécine, le méthotrexate, la vincristine, le tamoxifène, le BCNU, la streptozocine, la vincristine, le 5-fluorouracile ou des espéramicines.

15. Oligopeptide de BDB suivant l'une quelconque des revendications 1 à 4, en mélange avec un support.

16. Oligopeptide suivant la revendication 15, dans lequel ledit support est un support pharmaceutiquement acceptable.

**17.** Article manufacturé comprenant :

(a) un récipient ; et
(b) l'oligopeptide de BDB de l'une quelconque des revendications 1 à 5, logé dans ledit récipient ;
(c) une étiquette fixée audit récipient, ou un encart d'emballage incorporé audit récipient, mentionnant l'utilisation dudit oligopeptide pour le traitement thérapeutique ou la détection à des fins de diagnostic d'un cancer.

hCasp9-p12   A T P F Q E G L R
mCasp9-p12   A V P Y Q E G P R
xCasp9-p12   A T P V F S G E G
Smac / DIABLO   A V P I A Q K S E
HtrA2 / Omi   A V P S P P P A S
Reaper   A V A F Y I P D Q
Hid   A V P F Y L P E G
Grim   A I A Y F I P D Q
Sickle   A I P F F E E E H

## FIG._1

*FIG._2A*

*FIG._2B*

|  | Residue | | | | | | | | | | | | | | | |
|---|---|---|---|---|---|---|---|---|---|---|---|---|---|---|---|---|
|  | 1 | | 2 | | 3 | | 4 | | 5 | | 6 | | 7 | | 8 | |
| TARGET | AA | % | AA | % | AA | % | AA | % | AA | % | AA | % | AA | % | AA | % |
| **XIAP-BIR2** | A | 98 | E | 45 | A | 51 | V | 65 | D | 13 | W | 41 | A | 13 | A | 23 |
|  | L | 1 | V | 19 | G | 15 | I | 17 | P | 13 | E | 10 | E | 13 | V | 15 |
|  | S | 1 | T | 12 | V | 11 | D | 2.8 | G | 12 | G | 8.2 | W | 13 | W | 15 |
|  |  |  | Q | 11 | C | 6.2 | E | 2.8 | V | 9.2 | D | 8.2 | I | 13 | I | 15 |
|  |  |  | I | 8.5 | P | 6.2 | F | 2.8 | A | 9.2 | F | 6.2 | V | 13 | G | 11 |
|  |  |  | D | 4.3 | K | 3.1 | G | 2.8 | L | 7.9 | V | 5.2 | M | 13 | L | 10 |
|  |  |  | S | 0.7 | M | 3.1 | L | 2.8 | M | 7.9 | M | 4.1 | Q | 6.5 | P | 3.8 |
|  |  |  |  |  | S | 3.1 | W | 2.8 | F | 5.3 | A | 4.1 | G | 6.5 | T | 3.8 |
|  |  |  |  |  | R | 1 | A | 1.4 | W | 5.3 | P | 3.1 | L | 4.3 | S | 2.5 |
|  |  |  |  |  |  |  |  |  | I | 5.3 | C | 2.1 | T | 3.2 |  |  |
|  |  |  |  |  |  |  |  |  | Q | 2.6 | L | 2.1 | S | 2.2 |  |  |
|  |  |  |  |  |  |  |  |  | S | 2.6 | S | 2.1 |  |  |  |  |
|  |  |  |  |  |  |  |  |  | E | 2.6 | Y | 2.1 |  |  |  |  |
|  |  |  |  |  |  |  |  |  | Y | 2.6 | T | 1 |  |  |  |  |
|  |  |  |  |  |  |  |  |  | T | 1.3 |  |  |  |  |  |  |
| **XIAP-BIR3** | A | 100 | V | 47 | P | 93 | F | 66 | V | 17 | G | 17 | G | 21 | M | 37 |
|  |  |  | M | 9.7 | A | 3.7 | W | 12 | A | 16 | E | 16 | A | 16 | G | 26 |
|  |  |  | S | 11 | G | 2.5 | L | 9 | G | 13 | M | 12 | V | 12 | W | 15 |
|  |  |  | T | 6.1 | V | 1.2 | I | 6 | M | 13 | V | 12 | E | 8.2 | F | 7.3 |
|  |  |  | I | 4.9 |  |  | M | 3 | E | 10 | D | 8.1 | F | 8.2 | L | 4.9 |
|  |  |  | L | 4.9 |  |  | V | 3 | L | 7.6 | A | 6 | I | 8.2 | A | 3.7 |
|  |  |  | F | 4.9 |  |  | Y | 1.5 | F | 6.3 | T | 5 | M | 8.2 | T | 3.7 |
|  |  |  | R | 3.2 |  |  |  |  | I | 4.2 | L | 4.7 | W | 8.2 | V | 3.7 |
|  |  |  | A | 2.4 |  |  |  |  | N | 4.2 | S | 4.7 | P | 4.1 |  |  |
|  |  |  | E | 2.4 |  |  |  |  | T | 3.1 | F | 4 | L | 2.7 |  |  |
|  |  |  | Q | 2.4 |  |  |  |  | S | 2.8 | Q | 4 | S | 2.7 |  |  |
|  |  |  | G | 1.2 |  |  |  |  | Q | 2.1 | W | 4 |  |  |  |  |
|  |  |  |  |  |  |  |  |  | W | 2.1 | P | 2 |  |  |  |  |
| **ML-IAP-BIR** | A | 100 | V | 50 | P | 95 | F | 53 | E | 23 | E | 37 | C | 25 | V | 33 |
|  |  |  | I | 20 | V | 5 | W | 32 | G | 19 | L | 15 | W | 26 | E | 28 |
|  |  |  | A | 10 |  |  | I | 11 | A | 15 | A | 9.2 | E | 16 | C | 9.4 |
|  |  |  | T | 10 |  |  | M | 5.3 | I | 15 | G | 9.2 | V | 12 | M | 9.4 |
|  |  |  | L | 6.7 |  |  |  |  | L | 7.7 | S | 9.2 | A | 8.2 | W | 9.4 |
|  |  |  | S | 3.3 |  |  |  |  | M | 7.7 | D | 9.2 | F | 8.2 | P | 4.7 |
|  |  |  |  |  |  |  |  |  | W | 7.7 | R | 6.2 | L | 2.7 | L | 3.1 |
|  |  |  |  |  |  |  |  |  | V | 3.8 | T | 4.6 | S | 2.7 | S | 3.1 |

**FIG._3**

*FIG._4*

*FIG._5A*

*FIG._5B*

EP 1 590 666 B1

ML-IAP-BIR 79 PAFPGMGSEELRLASFYDWPLTAEVPPELLAAAGFFHTGHQDKVR 123
XIAP-BIR3 257 PRNPSMADYEARIFTFGTWIY--SVNKEQLARAGFYALGEGDKVK 299
XIAP-BIR2 155 PRNPAMYSEEARLKSFQNQPDYAHLTPRELASAGLYYTGIGDQVQ 199

ML-IAP-BIR 124 CFFCYGGLQSWKRGDDPWTEHAKWFPSCQFLLRSKGRDFVHSVQE 168
XIAP-BIR3 300 CFHCGGGLTDWKPSEDPWEQHAKWYPGCKYLLEQKGQEYINNIHL 344
XIAP-BIR2 200 CFCCGGKLKNWEPCDRAWSEHRRHFPNCFFVL 231

## FIG._5C

**FIG._6A**

**FIG._6B**

FIG._6C

FIG._7

*FIG._8*

FIG._9A

FIG._9B

1h (ppm)

EP 1 590 666 B1

A(Xaa)IAQKSE (SEQ ID NO:53) where Xaa is
(3S)-c-3-amino-1-carboxymethyl-caprolactame

## FIG._10A

[3R,6S,10R]-6-aminooctahydro-5-oxo-thiazolo
[3,2-a]azepine-3-carboxylic acid

## FIG._10B

[3R,6S,10R]-6-aminooctahydro-5-oxo-2,2-dimethyl-thiazolo
[3,2-a]azepine-3-carboxylic acid

## FIG._10C

[3R,6S,10R]-6-aminooctahydro-5-oxo-2,2-dimethyl-thiazolo
[3,2-a]azepine-3-carboxylic acid

## FIG._10D

[3R,6S,9R]-6-aminohexahydro-5-oxo-2,2-dimethyl-thiazolo
[3,2-a]pyridine-3-carboxylic acid

## FIG._10E

**FIG._11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02096930 A
- US 4275149 A
- US 5556762 A
- US 5750373 A
- US 4708871 A
- US 4833092 A
- US 5223409 A
- US 5403484 A
- US 5571689 A
- US 5663143 A
- WO 8403506 A
- WO 8403564 A
- WO 984547 A
- US 4933294 A
- WO 9105264 A
- US 5401638 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5712374 A
- US 5714586 A
- US 5739116 A
- US 5767285 A
- US 5770701 A
- US 5770710 A
- US 5773001 A
- US 5877296 A
- US 5053394 A
- WO 9321232 A
- WO 9411026 A
- US 5208020 A
- WO 9534683 A
- US 5627024 A
- US 5766905 A
- WO 9814277 A
- WO 9820169 A
- WO 9820159 A
- WO 9820036 A
- WO 9735196 A
- WO 9746251 A
- WO 9747314 A
- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- WO 8905859 A
- US 4399216 A
- DD 266710
- US 4946783 A
- US 5648237 A, Carter
- US 5789199 A, Joly
- US 5840523 A, Simmons
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 73657 A
- GB 2211504 A
- EP 117060 A
- EP 117058 A
- US 4767704 A
- US 4657866 A
- US 4927762 A

- US 4560655 A
- US 5122469 A
- WO 9003430 A
- WO 8700195 A
- US RE30985 E
- EP 0600517 A

- EP 616812 A
- WO 9607321 A
- US 4892538 A
- US 5283187 A
- WO 9325673 A

**Non-patent literature cited in the description**

- **THOMPSON et al.** *Science,* 1995, vol. 267, 1456-1462
- **DEVERAUX et al.** *J Clin Immunol,* 1999, vol. 19, 388-398
- **CROOK et al.** *J Virology,* 1993, vol. 67, 2168-2174
- **HINDS et al.** *Nat. Struct. Biol.,* 1999, vol. 6, 648-651
- **DEVERAUX et al.** *EMBO J.,* vol. 17, 2215-2223
- **VUCIC et al.** *Current Bio,* 2000, vol. 10, 1359-1366
- **CHAI et al.** *Nature,* 2000, vol. 406, 855-862
- **LIU et al.** *Nature,* 2000, vol. 408, 1004-1008
- **WU et al.** *Nature,* 2000, vol. 408, 1008-1012
- **DUCKETT et al.** *EMBO J.,* 1996, vol. 15, 2685-2694
- **GEYSEN et al.** *Proc. Natl. Acad. Sci. U.S.A.,* 1984, vol. 81, 3998-4002
- **GEYSEN et al.** *Proc. Natl. Acad. Sci. U.S.A.,* 1985, vol. 82, 178-182
- **GEYSEN et al.** *Synthetic Peptides as Antigens,* 1986, 130-149
- **GEYSEN et al.** *J. Immunol. Meth.,* 1987, vol. 102, 259-274
- **SCHOOFS et al.** *J. Immunol.,* 1988, vol. 140, 611-616
- **CWIRLA, S. E. et al.** *Proc. Natl. Acad. Sci. USA,* 1990, vol. 87, 6378
- **LOWMAN, H.B. et al.** *Biochemistry,* 1991, vol. 30, 10832
- **CLACKSON, T. et al.** *Nature,* 1991, vol. 352, 624
- **MARKS, J. D. et al.** *J. Mol. Biol.,* 1991, vol. 222, 581
- **KANG, A.S. et al.** *Proc. Natl. Acad. Sci. USA,* 1991, vol. 88, 8363
- **SMITH, G. P.** *Current Opin. Biotechnol.,* 1991, vol. 2, 668
- **SIAS et al.** *J. Immunol. Methods,* 1990, vol. 132, 73-80
- Cell cycle regulation, oncogenes, and antineoplastic drugs. The Molecular Basis of Cancer. WB Saunders, 1995
- **HINMAN et al.** *Cancer Research,* 1993, vol. 53, 3336-3342

- **LODE et al.** *Cancer Research,* 1998, vol. 58, 2925-2928
- **FRAKER et al.** *Biochem. Biophys. Res. Commun.,* 1978, vol. 80, 49-57
- Monoclonal Antibodies in Immunoscintigraphy. CRC Press, 1989
- **VITETTA et al.** *Science,* 1987, vol. 238, 1098
- **CHARI et al.** *Cancer Research,* vol. 52, 127-131
- **SCOTT, J.K. ; SMITH, G. P.** *Science,* 1990, vol. 249, 386
- **REN, Z-J. et al.** *Gene,* 1998, vol. 215, 439
- **ZHU, Z.** *CAN,* 1997, vol. 33, 534
- **JIANG, J. et al.** *can,* 1997, vol. 128, 44380
- **REN, Z-J. et al.** *CAN,* 1997, vol. 127, 215644
- **REN, Z-J.** *Protein Sci.,* 1996, vol. 5, 1833
- **EFIMOV, V. P. et al.** *Virus Genes,* 1995, vol. 10, 173
- **SMITH, G. P. ; SCOTT, J.K.** *Methods in Enzymology,* 1993, vol. 217, 228-257
- **LI et al.** *Mol Biotech.,* 1998, vol. 9, 187
- **PROCHIANTZ A.** *Curr. Opinion Neurobiol.,* 1996, vol. 5, 629-34
- **DEROSSI et al.** *Trends Cell Biol.,* 1998, vol. 8, 84-87
- **ARNT et al.** *Jour. Bio. Chem.,* 2002, vol. 277 (46), 44236-4424300
- In vitro diagnostic instrumentation. **KEATING et al.** Proceedings of SPIE. 2000, 128-137
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CUNNINGHAM ; WELLS.** *Science,* 1989, vol. 244, 1081-1085
- **CREIGHTON.** The Proteins. W.H. Freeman & Co,
- **CHOTHIA.** *J. Mol. Biol.,* 1976, vol. 150, 1
- Mammalian Cell Biotechnology: a Practical Approach. IRL Press, 1991
- **SHAW et al.** *Gene,* 1983, vol. 23, 315
- **GRAHAM ; VAN DER EB.** *Virology,* 1978, vol. 52, 456-457
- **VAN SOLINGEN et al.** *J. Bact.,* 1977, vol. 130, 946

- **HSIAO et al.** *Proc. Natl. Acad. Sci. (USA),* 1979, vol. 76, 3829
- **KEOWN et al.** *Methods in Enzymology,* 1990, vol. 185, 527-537
- **MANSOUR et al.** *Nature,* 1988, vol. 336, 348-352
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 154 (2), 737-742
- **VAN DEN BERG et al.** *Bio/Technology,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **BALLANCE et al.** *Biochem. Biophys. Res. Commun.,* 1983, vol. 112, 284-289
- **TILBURN et al.** *Gene,* 1983, vol. 26, 205-221
- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, vol. 269
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *Annals N.Y. Acad. Sci.,* 1982, vol. 383, 44-68
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- *EP,* vol. 36, 776
- **DEBOER et al.** *Proc. Natl. Acad. Sci. USA,* 1983, vol. 80, 21-25
- **HITZEMAN et al.** *J. Biol, Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEI et al.** *Nature,* 1979, vol. 281, 40-46
- **HAM et al.** *Meth. Enz.,* 1979, vol. 58, 44
- **BARNES et al.** *Anal. Biochem.,* 1980, vol. 102, 255
- **NG et al.** *Mol. Cancer Ther.,* 2002, vol. 12, 1051-1058
- **GUO et al.** *Blood,* 2002, vol. 99, 3419-3426
- **FULDA et al.** *Nature Medicine,* 2002, vol. 8, 808-815
- Chemotherapy Service. Williams & Wilkins, 1992
- **ANDERSON et al.** *Science,* 1992, vol. 256, 808-813
- **MARASCO et al.** *Proc. Natl. Acad. Sci. USA,* 1993, vol. 90, 7889-7893
- **SUN et al.** *Nature,* 1999, vol. 401, 818-822
- **SUN et al.** *J. Biol Chem.,* 2000, vol. 275, 33777-33781
- **SIDHU et al.** *Methods Enzymology,* 2000, vol. 328, 333-363
- **RIEDL et al.** *Cell,* 2001, vol. 104, 791-800
- **WU et al.** *Mol. Cell,* 2001, vol. 8, 95-104
- **KIPP et al.** *Biochemistry,* 2002, vol. 41, 7344-7349
- **SHUKER et al.** *Science,* 1996, vol. 274, 1531-1534
- **PAN et al.** *Science,* 1997, vol. 277, 815-818
- **VUCIC et al.** *J.Biol. Chem.,* 2002, vol. 277, 12275-12279